# EUROPEAN PATENT SPECIFICATION

(11) **EP 1 333 787 B1**
(45) Date of publication and mention of the grant of the patent: **23.12.2009**
(21) Application number: 01990900.1
(22) Date of filing: 08.11.2001
(51) Int. Cl.: A61F 11/00, A61F 2/82, A61F 2/00

(54) **IMPLANTABLE VASCULAR GRAFT**
IMPLANTIERBARE GEFÄSSPROTHESE
GREFFE VASCULAIRE IMPLANTABLE

(30) Priority: 15.11.2000 US 714854; 01.12.2000 US 728582; 22.12.2000 US 747094; 26.06.2001 US 891620
(43) Date of publication of application: 13.08.2003
(73) Proprietor: Endologix, Inc., Irvine, CA 92618 (US)
(72) Inventor: SHAOLIAN, Samuel, M., Newport Beach, CA 92660 (US); MADRID, Gilbert, Laguna Niguel, CA 92677 (US); NGUYEN, Thanh, Van, Irvine, CA 92604 (US); PHAM, Trinh, Van, Santa Ana, CA 92703 (US); ZENG, Frank, M., Irvine, CA 92614 (US)
(74) Representative: Lucking, David John
(86) International application number: PCT/US2001/047028
(87) International publication number: WO 2002/039888

(56) References cited:
- WO-A-00/33769
- US-A- 5 201 757
- US-A- 5 415 664
- US-A- 5 545 211
- US-A- 5 545 211
- US-A- 5 683 451
- US-A- 5 690 644
- US-A- 5 716 365
- US-A- 5 716 365
- US-A- 5 824 037
- US-A- 5 919 225

## Description

### Background of the invention

The present invention relates to an endoluminal vascular prosthesis, and in particular, to a self-expanding bifurcated prosthesis for use in the treatment of abdominal aortic aneurysms.

An abdominal aortic aneurysm is a sac caused by an abnormal dilation of the wall of the aorta, a major artery of the body, as it passes through the abdomen. The abdomen is that portion of the body which lies between the thorax and the pelvis. It contains a cavity, known as the abdominal cavity, separated by the diaphragm from the thoracic cavity and lined with a serous membrane, the peritoneum. The aorta is the main trunk, or artery, from which the systemic arterial system proceeds. It arises from the left ventricle of the heart, passes upward, bends over and passes down through the thorax and through the abdomen to about the level of the fourth lumbar vertebra, where it divides into the two common iliac arteries.

The aneurysm usually arises in the infrarenal portion of the diseased aorta, for example, below the kidneys. When left untreated, the aneurysm may eventually cause rupture of the sac with ensuing fatal hemorrhaging in a very short time. High mortality associated with the rupture led initially to transabdominal surgical repair of abdominal aortic aneurysms. Surgery involving the abdominal wall, however, is a major undertaking with associated high risks. There is considerable mortality and morbidity associated with this magnitude of surgical intervention, which in essence involves replacing the diseased and aneurysmal segment of blood vessel with a prosthetic device which typically is a synthetic tube, or graft, usually fabricated of Polyester, Urethane, DACRON[], TEFLON[], or other suitable material.

To perform the surgical procedure requires exposure of the aorta through an abdominal incision which can extend from the rib cage to the pubis. The aorta must be closed both above and below the aneurysm, so that the aneurysm can then be opened and the thrombus, or blood clot, and arteriosclerotic debris removed. Small arterial branches from the back wall of the aorta are tied off. The DACRON[] tube, or graft, of approximately the same size of the normal aorta is sutured in place, thereby replacing the aneurysm. Blood flow is then reestablished through the graft. It is necessary to move the intestines in order to get to the back wall of the abdomen prior to clamping off the aorta.

If the surgery is performed prior to rupturing of the abdominal aortic aneurysm, the survival rate of treated patients is markedly higher than if the surgery is performed after the aneurysm ruptures, although the mortality rate is still quite high. If the surgery is performed prior to the aneurysm rupturing, the mortality rate is typically slightly less than 10%. Conventional surgery performed after the rupture of the aneurysm is significantly higher, one study reporting a mortality rate of 66.5%. Although abdominal aortic aneurysms can be detected from routine examinations, the patient does not experience any pain from the condition. Thus, if the patient is not receiving routine examinations, it is possible that the aneurysm will progress to the rupture stage, wherein the mortality rates are significantly higher.

Disadvantages associated with the conventional, prior art surgery, in addition to the high mortality rate include the extended recovery period associated with such surgery; difficulties in suturing the graft, or tube, to the aorta; the loss of the existing aorta wall and thrombosis to support and reinforce the graft; the unsuitability of the surgery for many patients having abdominal aortic aneurysms; and the problems associated with performing the surgery on an emergency basis after the aneurysm has ruptured. A patient can expect to spend from one to two weeks in the hospital after the surgery, a major portion of which is spent in the intensive care unit, and a convalescence period at home from two to three months, particularly if the patient has other illnesses such as heart, lung, liver, and/or kidney disease, in which case the hospital stay is also lengthened. The graft must be secured, or sutured, to the remaining portion of the aorta, which may be difficult to perform because of the thrombosis present on the remaining portion of the aorta. Moreover, the remaining portion of the aorta wall is frequently friable, or easily crumbled.

Since many patients having abdominal aortic aneurysms have other chronic illnesses, such as heart, lung, liver, and/or kidney disease, coupled with the fact that many of these patients are older, the average age being approximately 67 years old, these patients are not ideal candidates for such major surgery.

More recently, a significantly less invasive clinical approach to aneurysm repair, known as endovascular grafting, has been developed. Parodi, et al. provide one of the first clinical descriptions of this therapy. Parodi, J.C., et al., "Transfemoral Intraluminal Graft implantation for Abdominal Aortic Aneurysms," 5 Annals of Vascular Surgery 491 (1991). Endovascular grafting involves the transluminal placement of the prosthetic arterial graft within the lumen of the artery.

In general, transluminally implantable prostheses adapted for use in the abdominal aorta comprise a tubular wire cage surrounded by a tubular PTFE or Dacron sleeve. Both balloon expandable and self expandable support structures have been proposed. Endovascular grafts adapted to treat both straight segments and bifurcation aneurysms have also been proposed. Our earlier patent application, no. WO00133769 discloses such an implantable device with the features of the preamble of claim 1.

Notwithstanding the foregoing, there remains a need for a structurally simple, easily deployable transluminally implantable endovascular prosthesis, with a support structure adaptable to span either a straight or bifurcated aortic aneurysm. Preferably, the tubular prosthesis can be self expanded at the site to treat the abdominal aortic aneurysm, and exhibits flexibility to accommodate nonlinear anatomies and normal anatomical movement.

There is provided in accordance with one aspect of the present invention, a tubular wire support for a combination with a sheath to produce a flexible bifurcated endoluminal prosthesis.

The wire support comprises a main body support structure having a proximal end, a distal end and a central lumen extending therethrough, the support structure comprising at least a first and second axially adjacent tubular segments. Each segment comprises a plurality of wall struts connected by proximal and distal bends.

A first branch support structure, having a proximal end, a distal end and a central lumen therethrough is connected to the main body support structure. A second branch support structure, having a proximal end, a distal end and a central lumen extending therethrough, is connected to the main body support structure. At least two sliding links are provided inbetween the first and second segments on the main body support structure and at least one lock is provided on a wall strut for limiting axial movement of a sliding link along that strut.

In one application, the lock comprises a loop formed into the strut, for limiting the effective length of travel of the sliding link along that strut. At least about 30%, and in some devices at least 50%, and in other devices 100% of the links inbetween the first and second segments are provided with a sliding link configuration. At least about 30%, and in some applications at least about 50% of the sliding links are provided with a lock on the corresponding strut.

Further features and advantages of the present invention will become apparent to those of ordinary skill in the art in view of the disclosure herein, when considered together with the attached drawings and claims.

### Brief Description of the Drawings

Not all figures show the lock on a wall strut which characterises the invention (as in claim 1).
Figure 1 is a schematic representation of a straight segment vascular prosthesis in accordance with the present invention, positioned within a symmertric abdominal aortic aneurysm.
Figure 2 is an exploded view of an endoluminal vascular prosthesis in accordance with the present invention, showing a self expandable wire support structure separated from an outer tubular sleeve.
Figure 3 is a plan view of a formed wire useful for rolling about an axis into a multi-segment support structure in accordance with the present invention.
Figure 4 is an enlarged detail view of a portion of the formed wire illustrated in Figure 3.
Figure 5 is a schematic view of a porition of a wire cage wall, illustrating folded link connections between adjacent apexes.
Figure 6 is an exploded view of two opposing apexes dimensioned for one embodiment of the folded link connection of the present invention.
Figure 7 is an enlarged view of a folded link, taken along the lines 7-7 in Figure 5.
Figure 8 is a cross-sectional view taken along the line 8-8 in Figure 7.

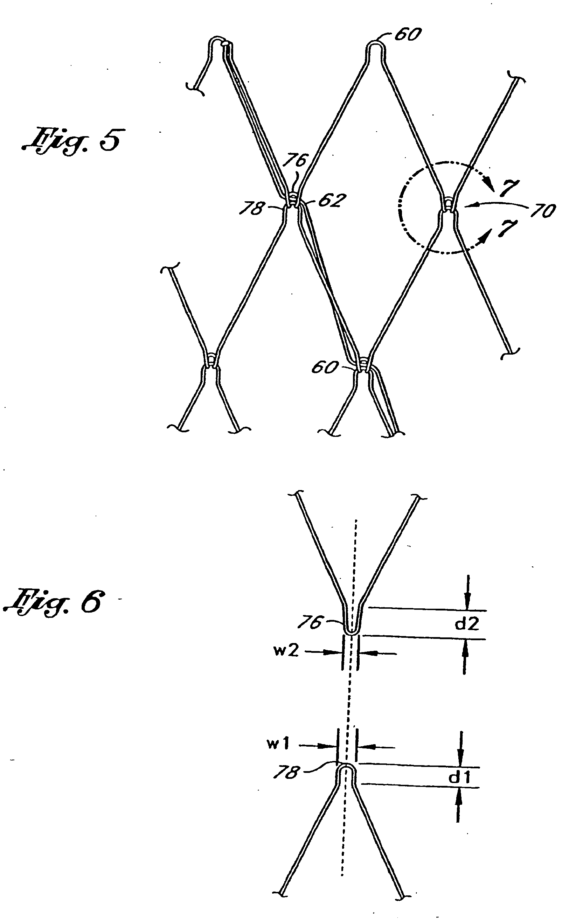

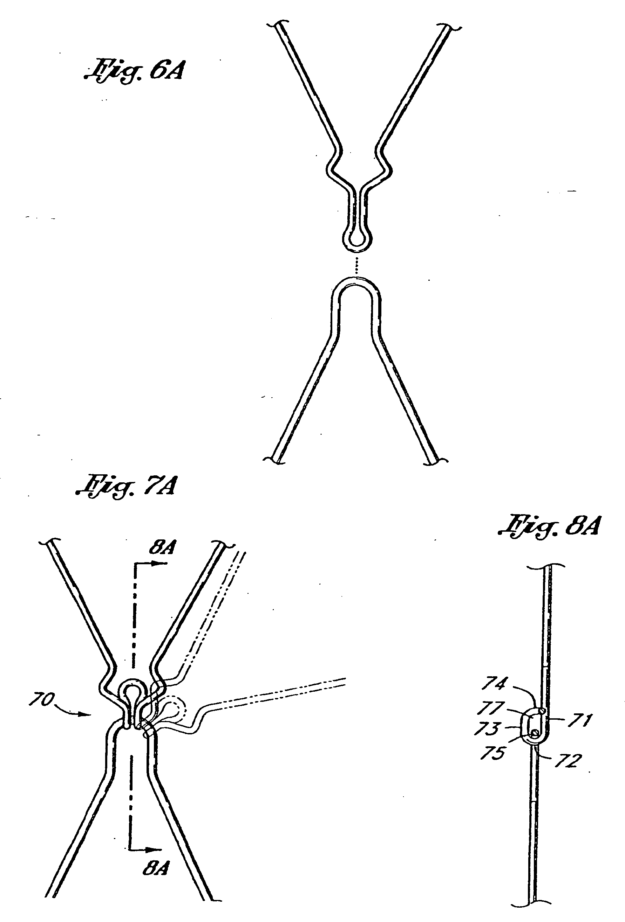

Figures 6A, 7A, 8A, 7B, 8B, 7C, and 7D illustrate alternate embodiments of a folded link constructed from an opposing apex pair.

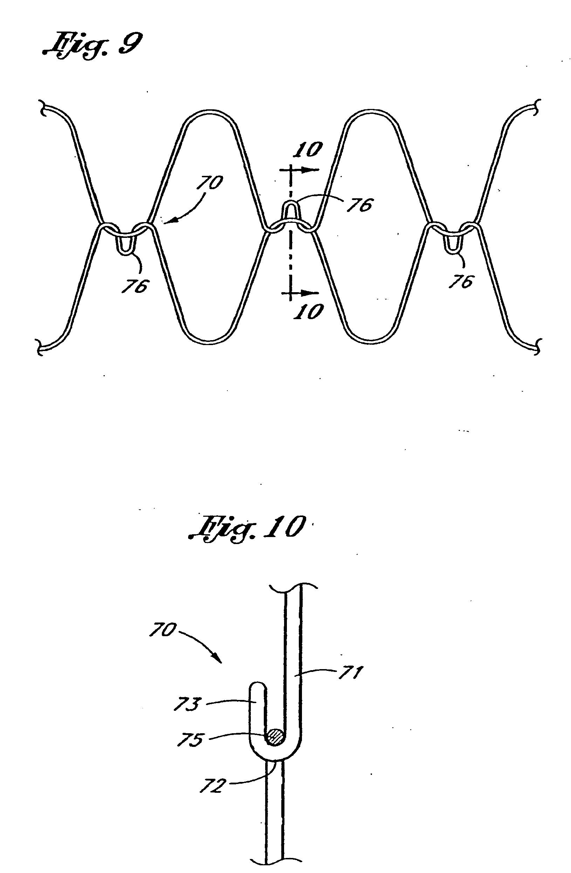

Figure 9 is a partial view of a junction between two adjacent tubular segments, illustrating oppositely oriented folded links in accordance with the present invention.
Figure 10 is a cross-section taken along the line 10-10 in Figure 9.

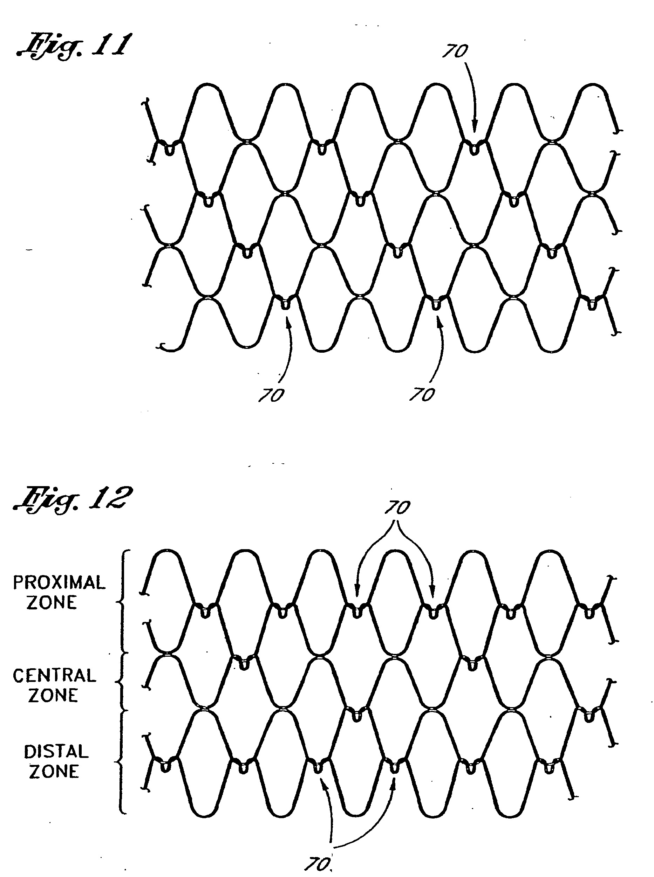

Figure 11 is a schematic view of a portion of a wall of a graft, laid out flat, illustrating an alternating folded link pattern.

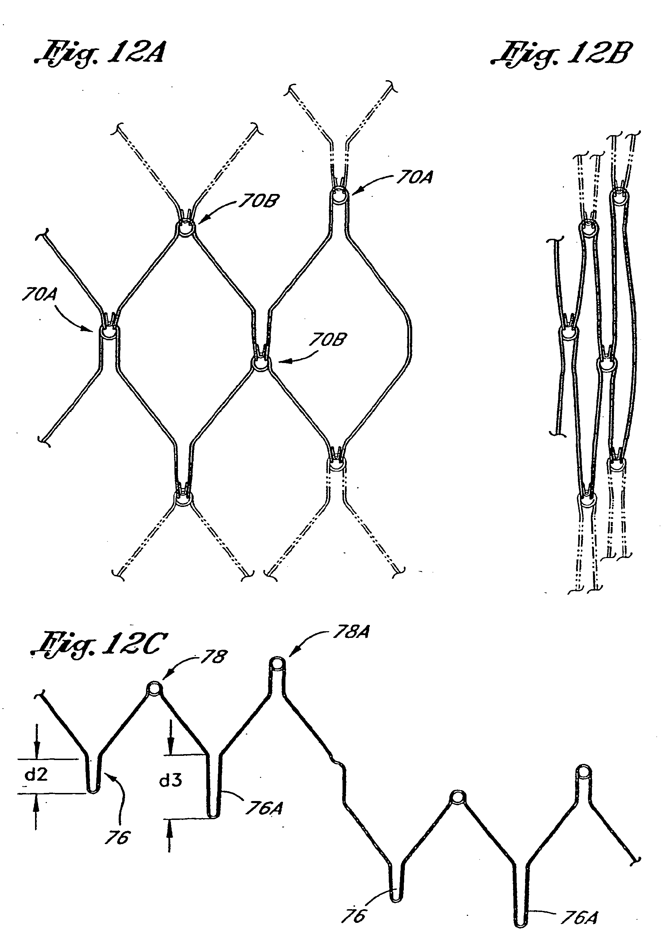

Figure 12 is a wall pattern as in Figure 11, illustrating a multi-zone folded link pattern.
Figures 12A through 12C illustrate an alternate wall pattern, which permits axially staggered links between adjacent graft segments.

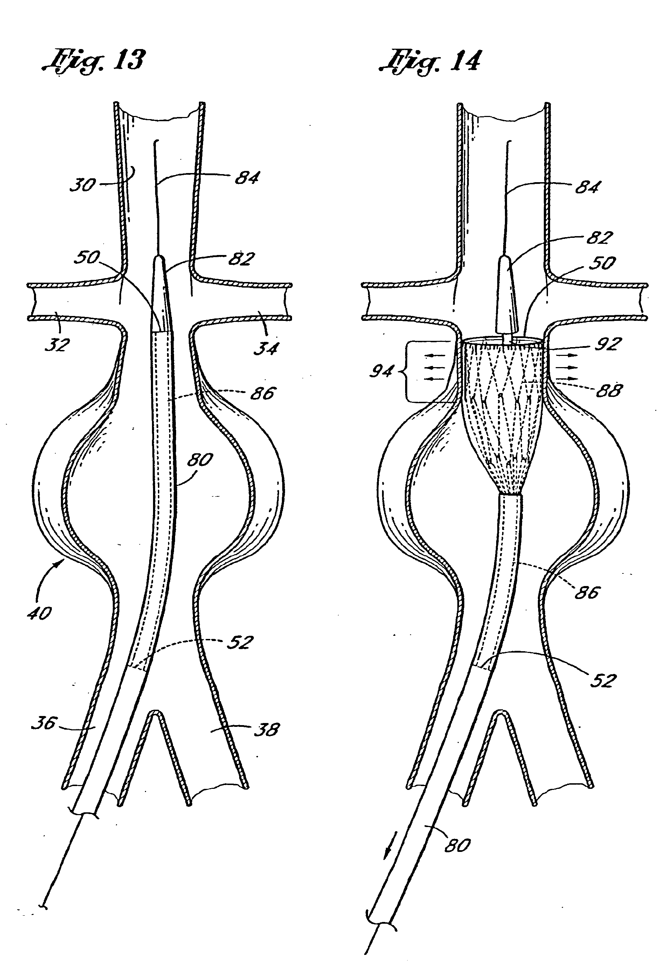

Figure 13 is a schematic illustration of a straight segment delivery catheter in accordance with the present invention, positioned within an abdominal aortic aneurysm.
Figure 14 is an illustration as in Figure 13, with the straight segment endoluminal prosthesis partially deployed from the delivery catheter.

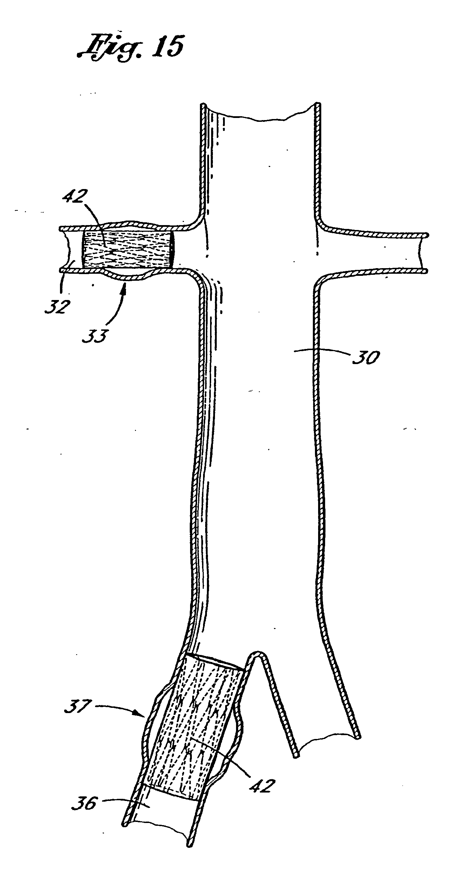

Figure 15 is a schematic representation of the abdominal aortic anatomy, with an endoluminal vascular prostheses of the present invention positioned within each of the right renal artery and the right common iliac.

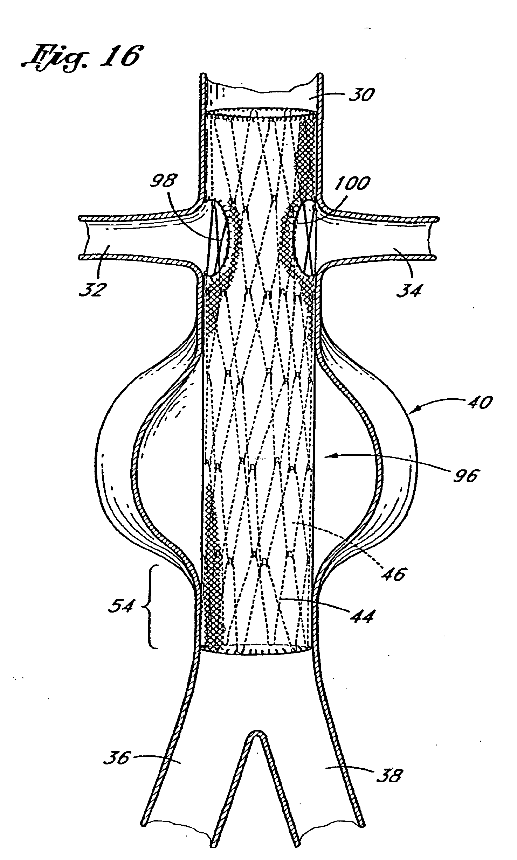

Figure 16 is a schematic representation of a straight segment graft in accordance with a further embodiment of the present invention, with side openings to permit renal perfusion.

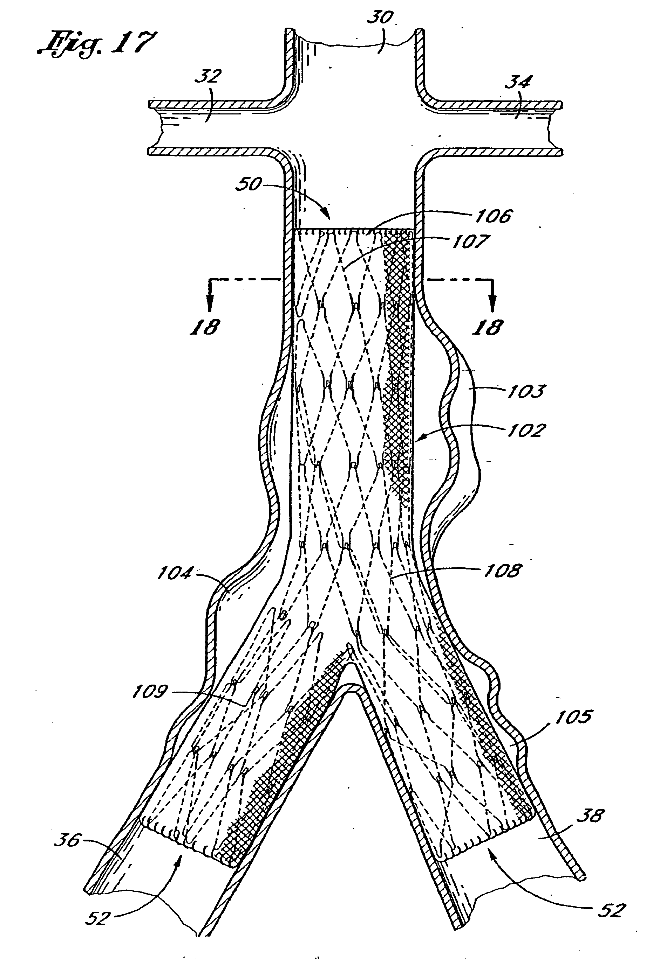

Figure 17 is a schematic representation of a bifurcated vascular prosthesis in accordance with the present invention, positioned at the bifurcation between the abdominal aorta and the right and left common iliac arteries.

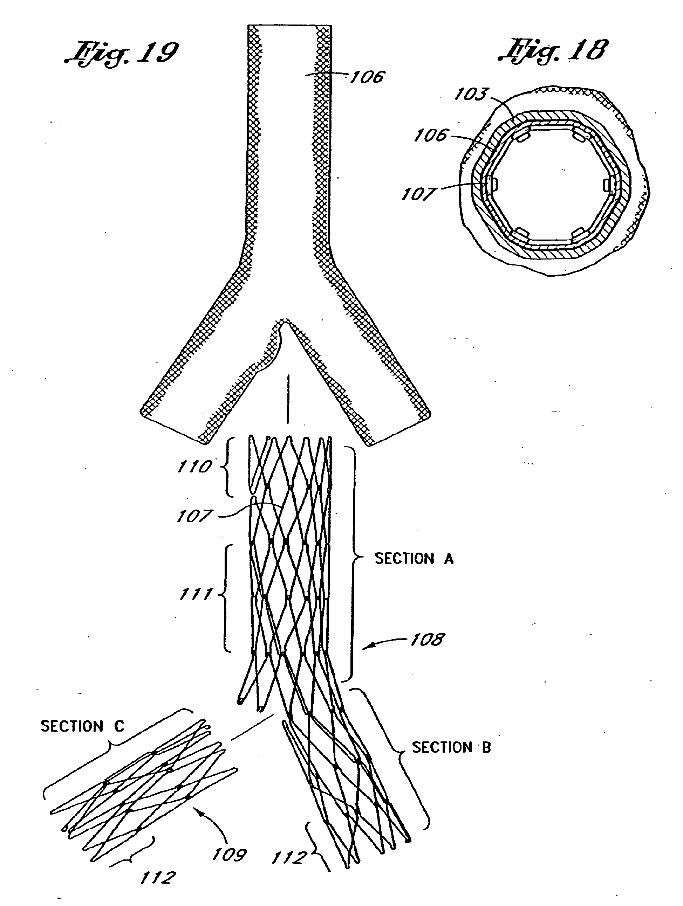

Figure 18 is a cross-sectional view of the implanted graft taken along the lines 18-18 of Figure 17.
Figure 19 is an exploded view of the bifurcated vascular prosthesis in accordance with the present invention, showing a two-part self expandable wire support structure separated from an outer tubular sleeve.

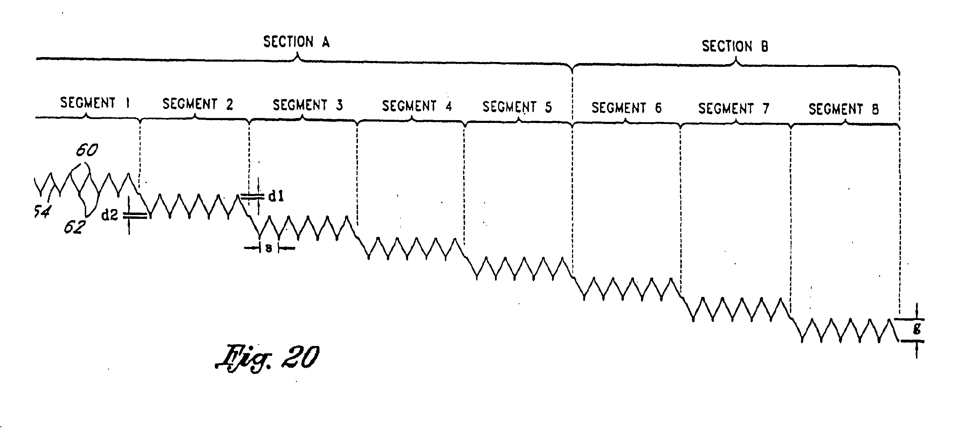

Figure 20 is a plan view of formed wire useful for rolling about an axis into an aortic trunk segment and a first iliac branch segment support structure in accordance with the present invention.

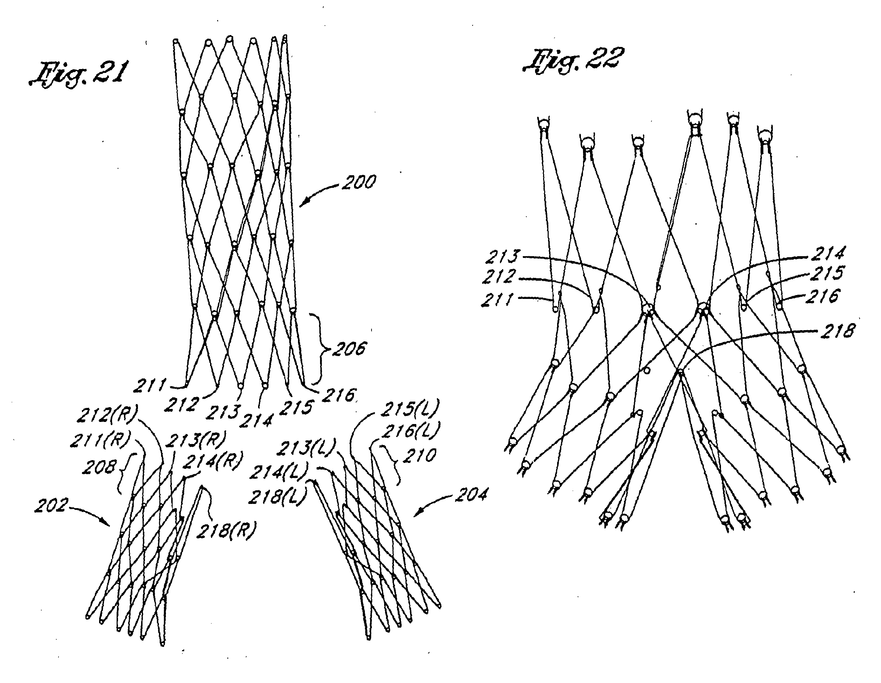

Figure 21 is a schematic representation of another embodiment of the wire support structure for the bifurcated vascular prosthesis of the present invention, showing a main body support structure and separate branch support structures.
Figure 22 is a schematic representation of the three-part wire support structure as in Figure 21, illustrating -the sliding articulation between the branch supports and the main body support.

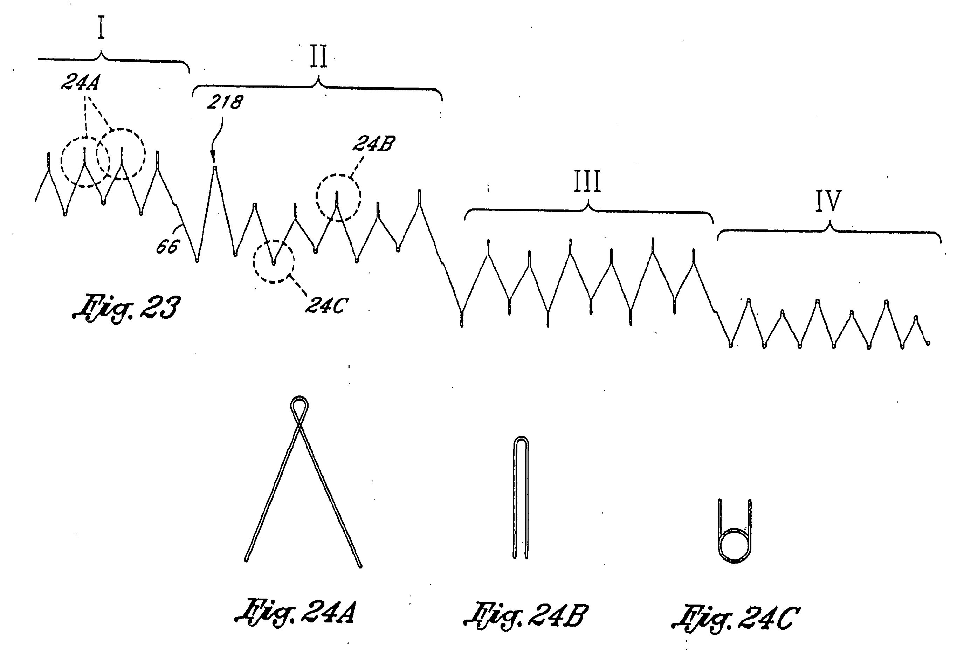

Figure 23 is a plan view of formed wire useful for rolling about an axis to form a branch support structure in accordance with the three-part support embodiment of the present invention shown in Figure 21.
Figures 24A, 24B and 24C are enlargements of the apexes delineated by lines A, B and C, respectively, in Figure 23.

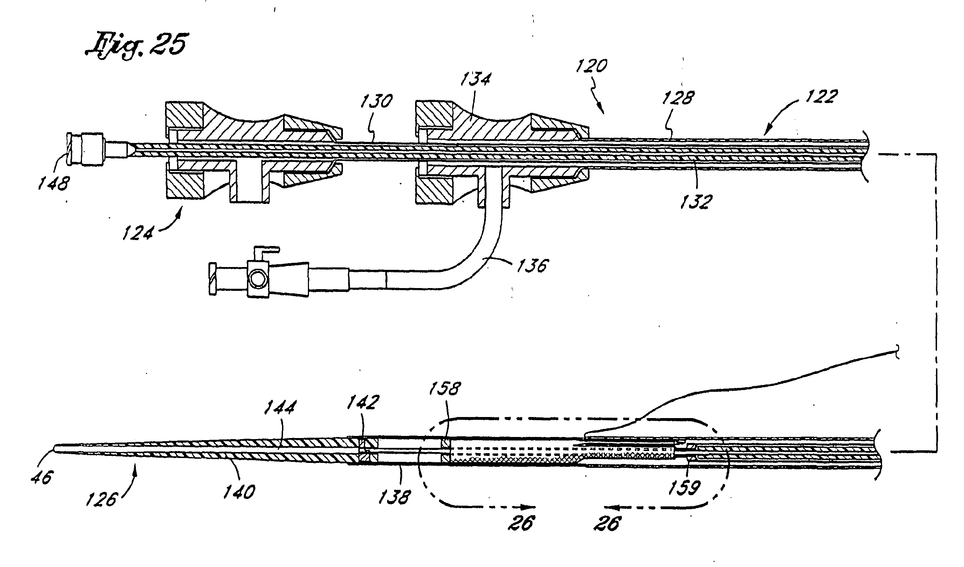

Figure 25 is side elevational cross-section of a bifurcation graft delivery catheter in accordance with the present invention.

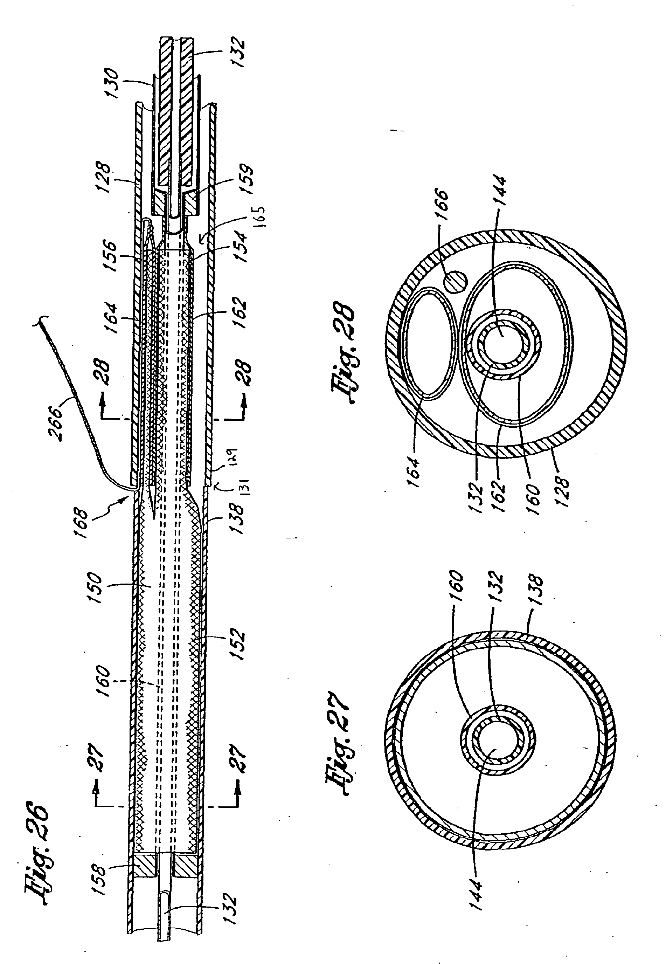

Figure 26 is an enlargement of the portion delineated by the line 26-26 in Figure 25.
Figure 27 is a cross-section taken along the line 27-27 in Figure 26.
Figure 28 is a cross-section taken along the line 28-28 in Figure 26.

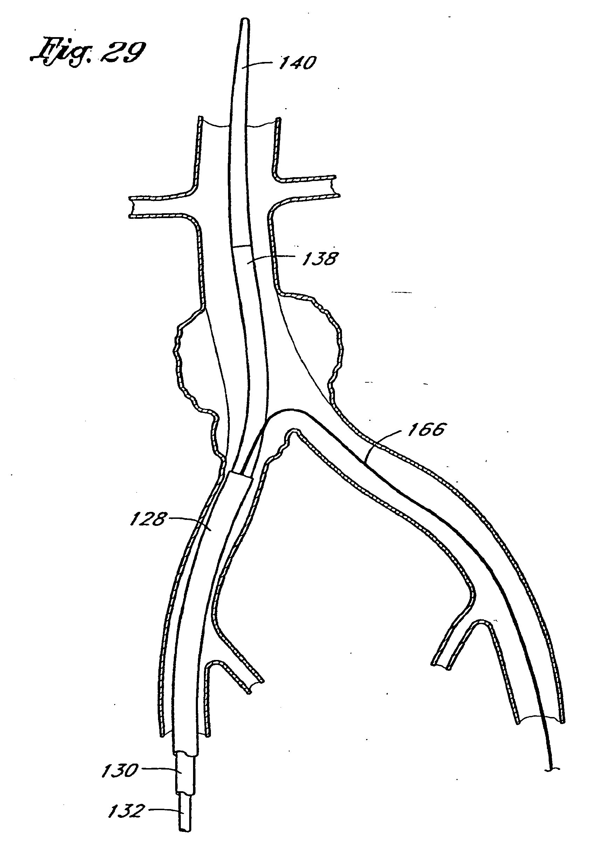

Figure 29 is a schematic representation of a bifurcated graft deployment catheter of the present invention, positioned within the ipsilateral iliac and the aorta, with the contralateral guidewire positioned within the contralateral iliac.

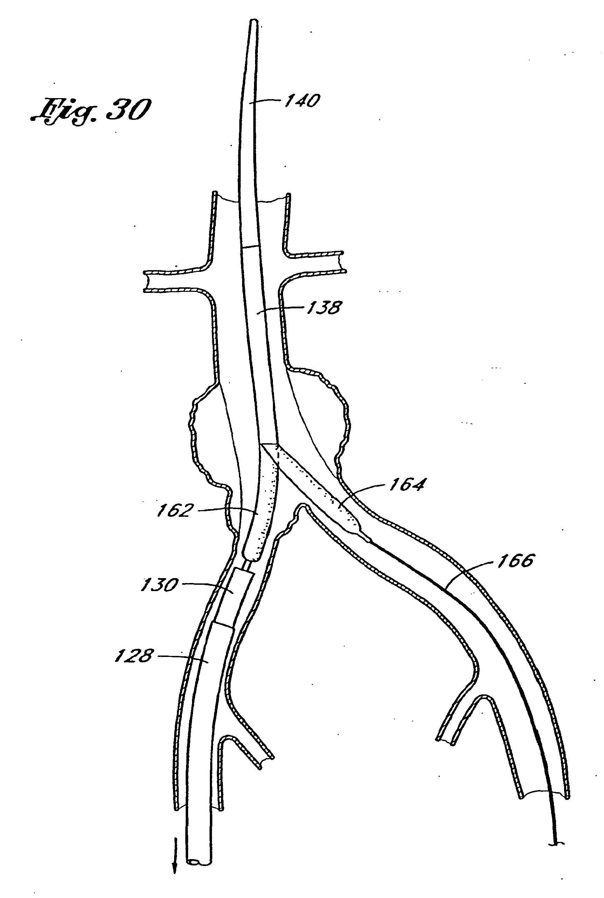

Figure 30 is a schematic representation as in Figure 29, with the outer sheath proximally retracted and the compressed iliac branches of the graft moving into position within the iliac arteries.

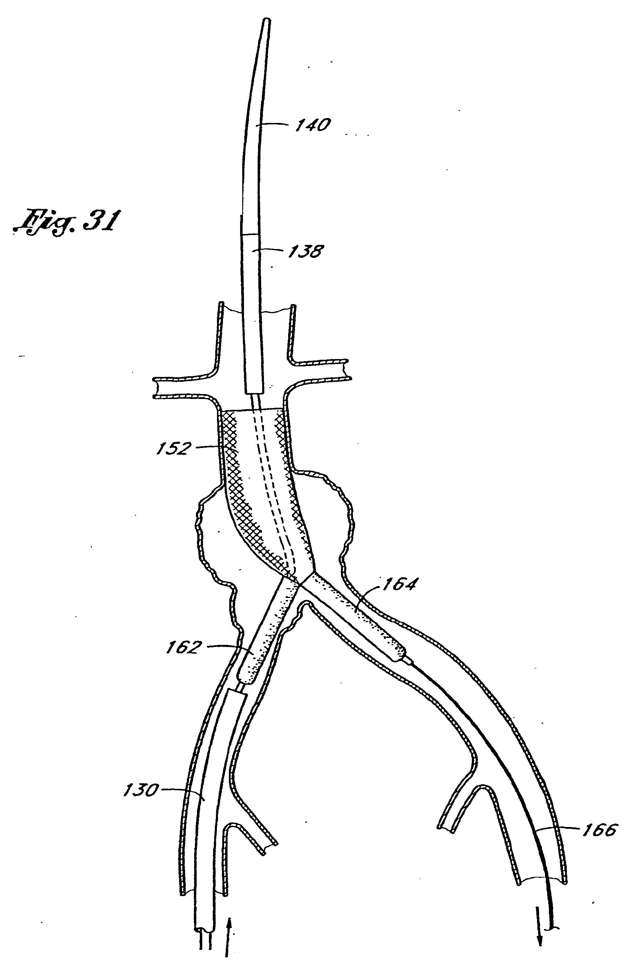

Figure 31 is a schematic representation as in Figure 30, with the compressed iliac branches of the graft within the iliac arteries, and the main aortic trunk of the graft deployed within the aorta.

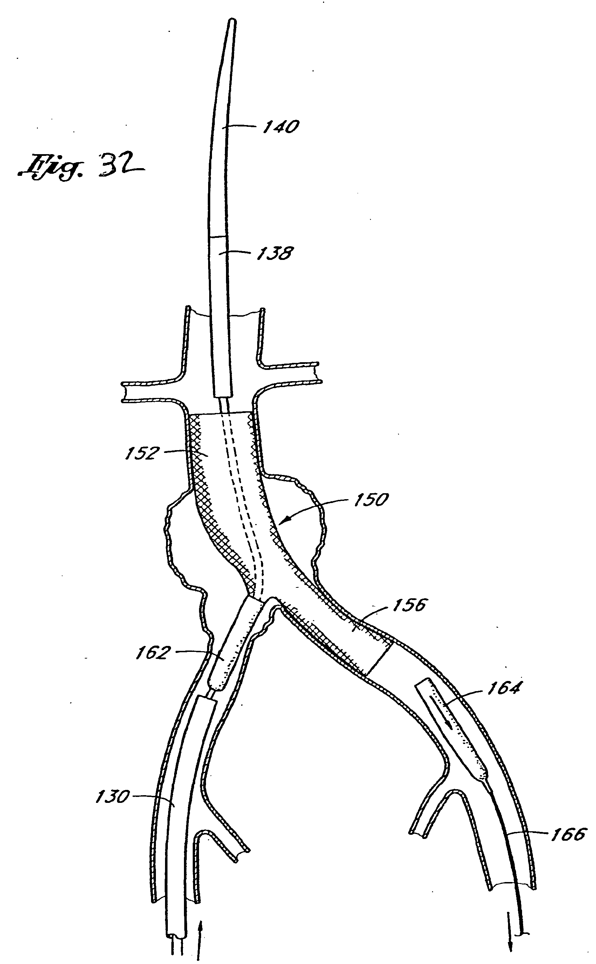

Figure 32 is a schematic representation as in Figure 31, with the contralateral iliac branch of the graft deployed.

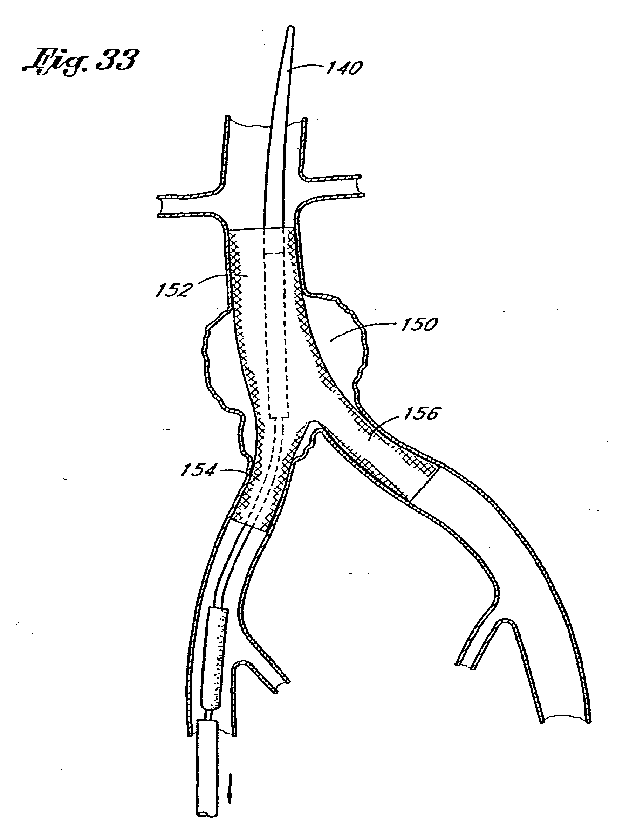

Figure 33 is a schematic representation as in Figure 32, following deployment of the ipsilateral branch of the graft.

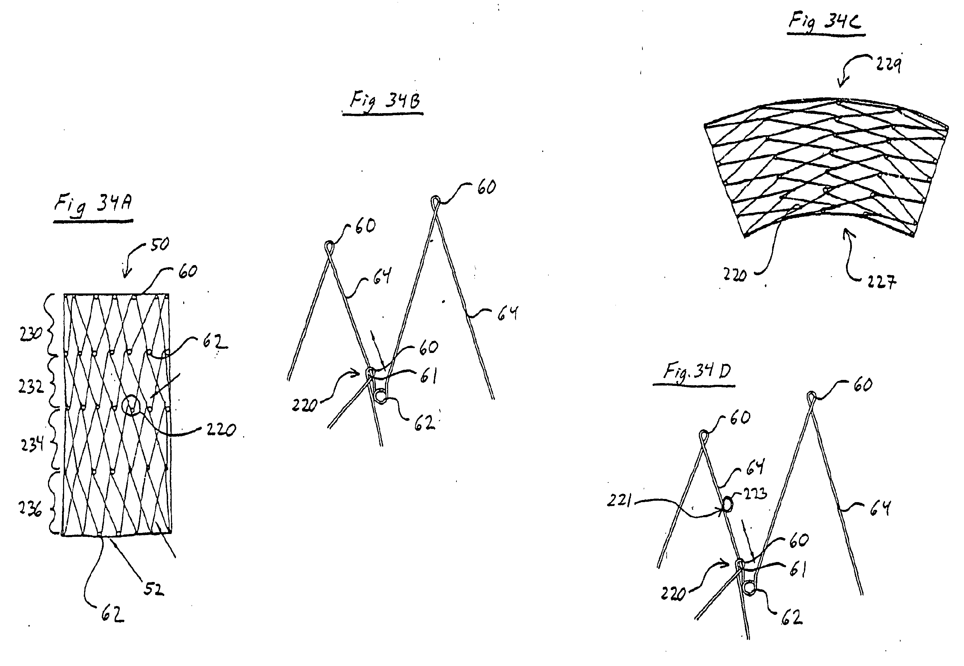

Figure 34A is a side elevational view of a flexible straight graft in accordance with the present invention.
Figure 34B is a detail view of a sliding link in the embodiment of Figure 34a.
Figure 34C is a side elevational view of the graft of Figure 34a, having a radius of curvature.
Figure 34D is a detail view as in Figure 34b, illustrating a lock for limiting axial compression of the sliding link.

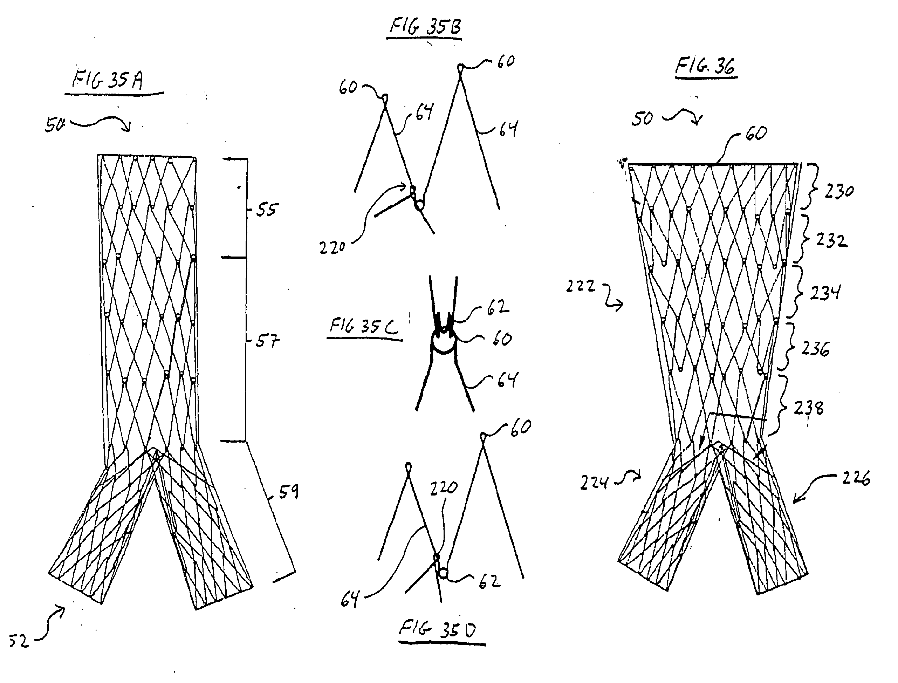

Figure 35A is a side elevational view of a bifurcated graft which includes sliding links in accordance with the present invention.
Figure 35B is a detail view of a sliding link in a proximal portion of the graft of Figure 35a.
Figure 35C is a detail view of a folded link utilized in an intermediate zone of the graft of Figure 35a.
Figure 35D is a detail view of a sliding link utilized in the iliac branches of the graft of Figure 35a.
Figure 36 is a side elevational view of a tapered bifurcated graft in accordance with the present invention.

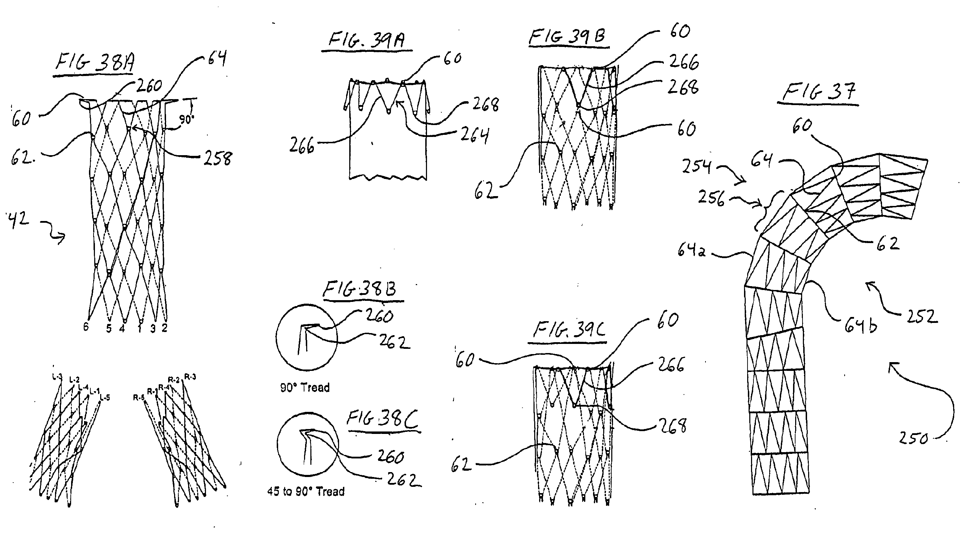

Figure 37 is a schematic side elevational view of a graft having short graft sections on one side to facilitate a curve.
Figure 38A illustrates a bifurcation or straight segment graft having a plurality of proximal barbs.
Figure 38B is a detailed view of a barb of the type illustrated in Figure 38a.
Figure 38C is an alternate barb configuration.
Figure 39A is a fractional side elevational view of a graft assembly having a partial exoskeleton.
Figure 39B is a partial side elevational view of an alternate exoskeleton configuration.
Figure 39C is a partial side elevational view of a second alternate partial exoskeleton configuration.

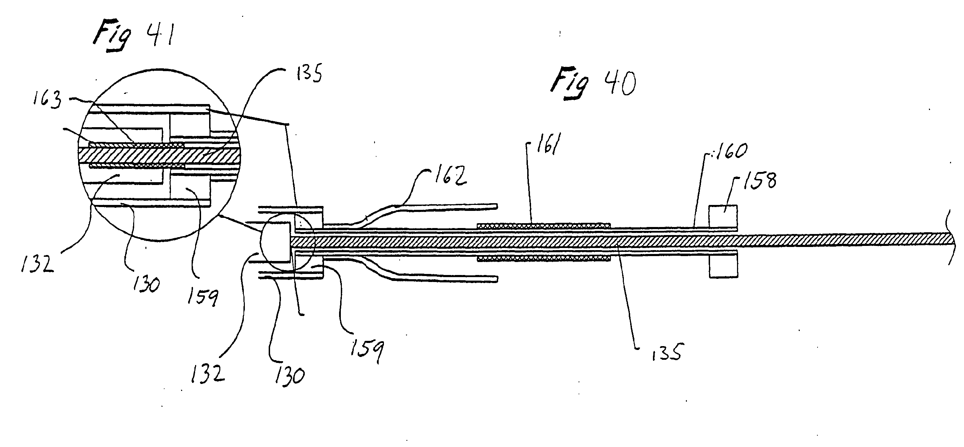

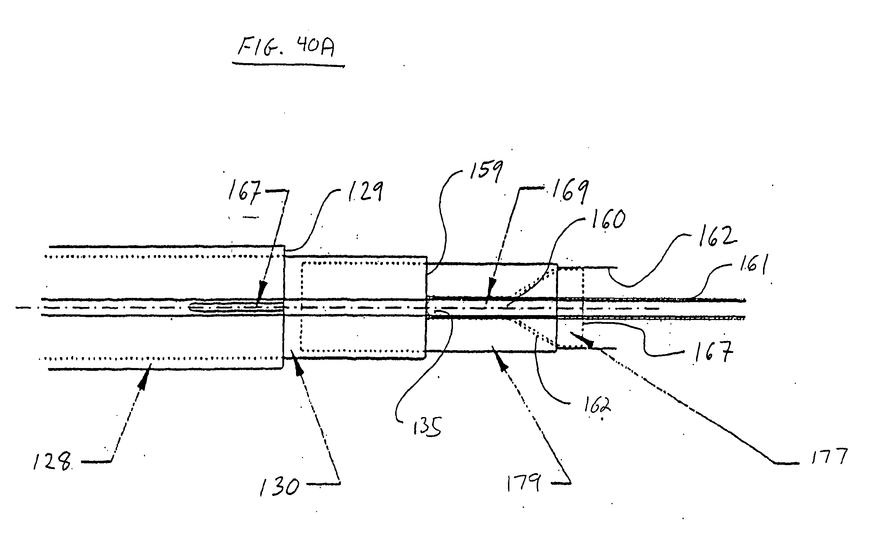

Figure 40 is a fragmentary side elevational view of an enhanced flexibility embodiment of the present invention.
Figure 40A is a fragmentary side elevational view of a further feature of the deployment catheter in accordance with the present invention.
Figure 41 is an enlarged detail view taken along along line 41-41 of Figure 40.

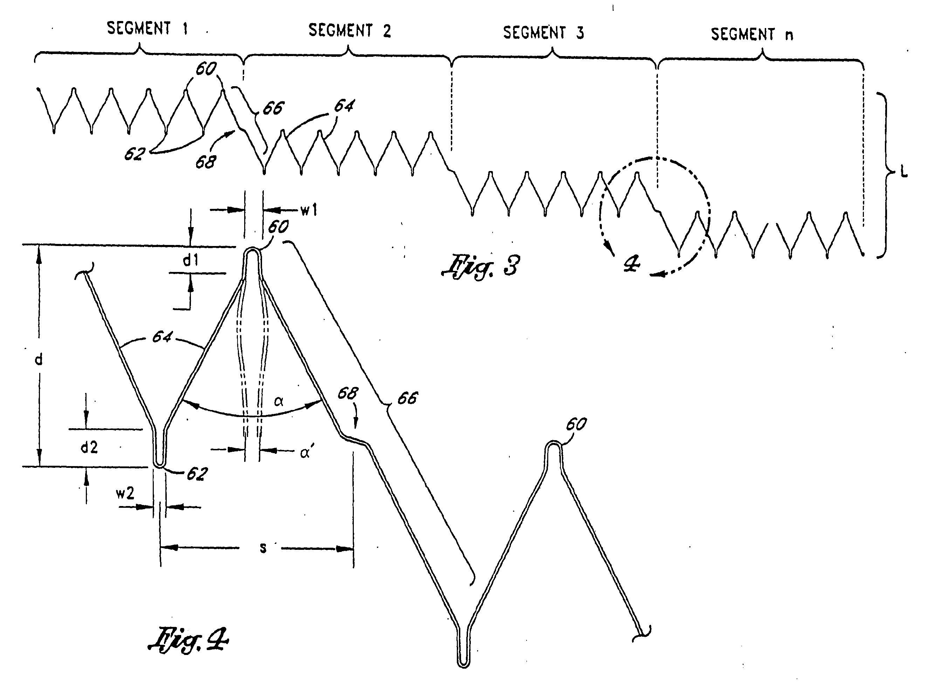

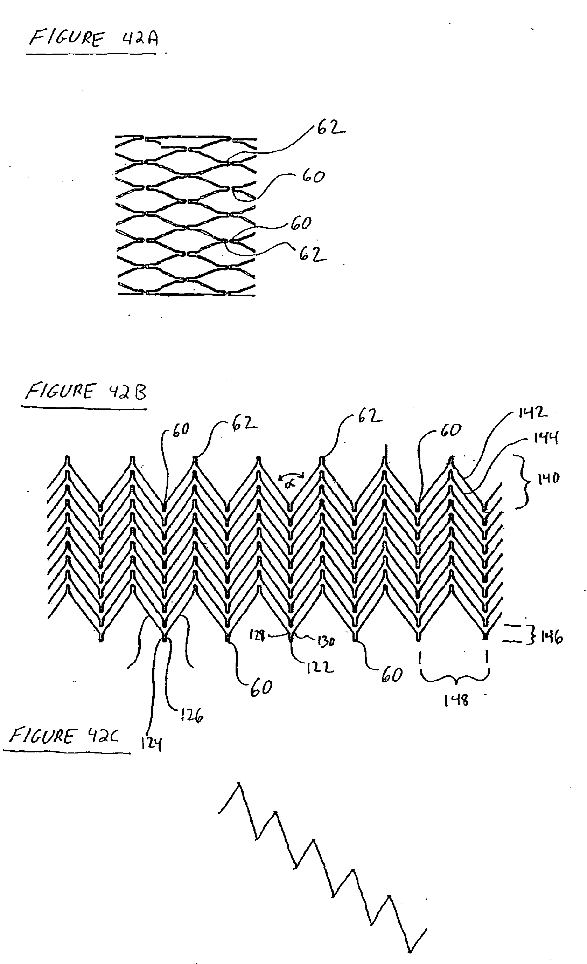

Figures 42A-D show alternative zig-zag wire or laser cut sheet support configurations that vary from the support illustrated in Figure 3.

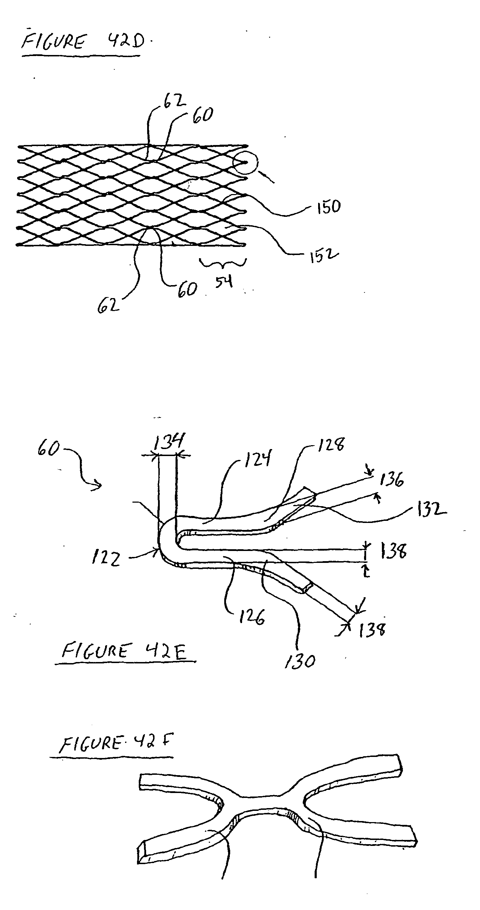

Figure 42E is a detail view of a bend identified in Figure 42D, illustrating a varied filament width feature of the invention.
Figure 42F is a detailed view of a modified link between adjacent segments.

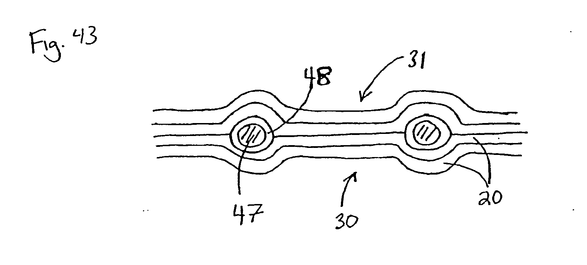

Figure 43 is a cross-sectional view taken through a portion of a prosthesis in which a wire support is embedded in an ePTFE wall.

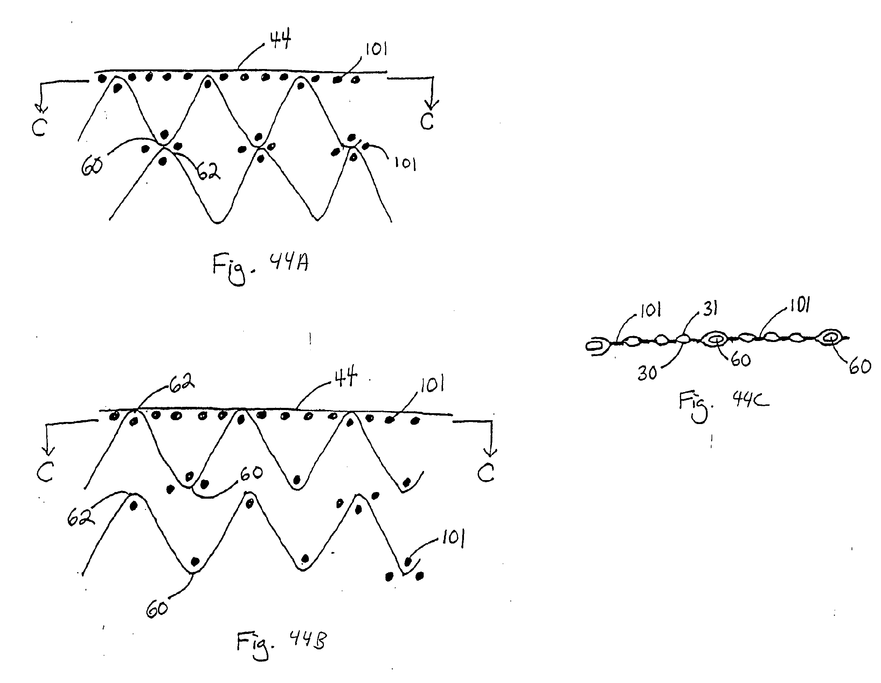

Figures 44A and B are schematic views of a portion of a wall of a graft, laid out flat, illustrating two alternative wire support configurations having spot welds between lumenal and exterior layers of a polymeric membrane.
Figure 44C is a cross-sectional view through the wall taken along line C-C in Figures 44A and B.

### Detailed Description of the Preferred Embodiment

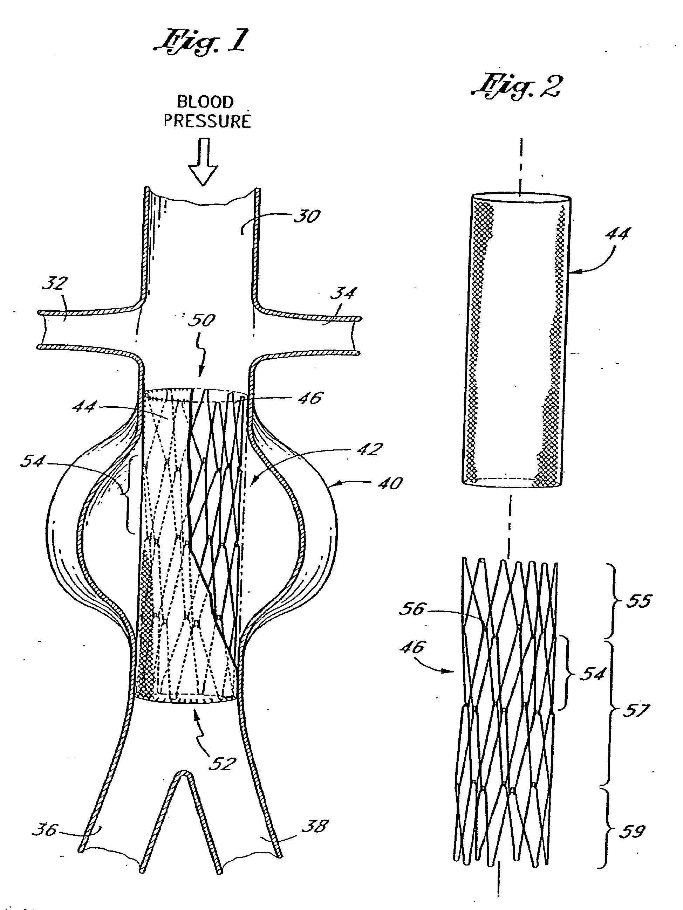

Referring to Figure 1, there is disclosed a schematic representation of the abdominal part of the aorta and its principal branches. In particular, the abdominal aorta 30 is characterized by a right renal artery 32 and left renal artery 34. The large terminal branches of the aorta are the right and left common iliac arteries 36 and 38. Additional vessels (e.g., second lumbar, testicular, inferior mesenteric, middle sacral) have been omitted for simplification. A generally symmetrical aneurysm 40 is illustrated in the infrarenal portion of the diseased aorta. An expanded straight segment endoluminal vascular prosthesis 42, in accordance with the present invention, is illustrated spanning the aneurysm 40.

The endoluminal vascular prosthesis 42 includes a polymeric sleeve 44 and a tubular wire support 46, which are illustrated *in situ* in Figure 1. The sleeve 44 and wire support 46 are more readily visualized in the exploded view shown in Figure 2. The endoluminal prosthesis 42 illustrated and described herein depicts an embodiment in which the polymeric sleeve 44 is situated concentrically outside of the tubular wire support 46. However, other embodiments may include a sleeve situated instead concentrically inside the wire support or on both of the inside and the outside of the wire support. Alternatively, the wire support may be embedded within a polymeric matrix which makes up the sleeve. Regardless of whether the sleeve 44 is inside or outside the wire support 46, the sleeve may be attached to the wire support by any of a variety of means, including laser bonding, adhesives, clips, sutures, dipping or spraying or others, depending upon the composition of the sleeve 44 and overall graft design.

The polymeric sleeve 44 may be formed from any of a variety of synthetic polymeric materials, or combinations thereof, including PTFE, PE, PET, Urethane, Dacron, nylon, polyester or woven textiles. Preferably, the sleeve material exhibits relatively low inherent elasticity, or low elasticity out to the intended enlarged diameter of the wire cage 46. The sleeve material preferably has a thin profile, such as no larger than about 0.002 inches to about 0.005 inches.

In a preferred embodiment of the invention, the material of sleeve 44 is sufficiently porous to permit ingrowth of endothelial cells, thereby providing more secure anchorage of the prosthesis and potentially reducing flow resistance, sheer forces, and leakage of blood around the prosthesis. Porosity in polymeric sleeve materials may be estimated by measuring water permeability as a function of hydrostatic pressure, which will preferably range from about 3 to 6 psi.

The porosity characteristics of the polymeric sleeve 44 may be either homogeneous throughout the axial length of the prosthesis 42, or may vary according to the axial position along the prosthesis 42. For example, referring to Figures 1 and 2, different physical properties will be called upon at different axial positions along the prosthesis 42 in use. At least a proximal portion 55 and a distal portion 59 of the prosthesis 42 will seat against the native vessel wall, proximally and distally of the aneurysm. In these proximal and distal portions, the prosthesis preferably encourages endothelial growth, or, at least, permits endothelial growth to infiltrate portions of the prosthesis in order to enhance anchoring and minimize leakage. A central portion 57 of the prosthesis spans the aneurysm, and anchoring is less of an issue. Instead, maximizing lumen diameter and minimizing blood flow through the prosthesis wall become primary objectives. Thus, in a central zone 57 of the prosthesis 42, the polymeric sleeve 44 may either be nonporous, or provided with pores of relatively lower porosity

A multi-zoned prosthesis 42 may also be provided in accordance with the present invention by positioning a tubular sleeve 44 on a central portion 57 of the prosthesis, such that it spans the aneurysm to be treated, but leaving a proximal attachment zone 55 and a distal attachment zone 59 of the prosthesis 42 having exposed wires from the wire support 46. In this embodiment, the exposed wires 46 are positioned in contact with the vessel wall both proximally and distally of the aneurysm, such that the wire, over time, may become embedded in cell growth on the interior surface of the vessel wall.

In one embodiment of the prosthesis 42, the sleeve 44 and/or the wire support 46 is tapered, having a relatively larger expanded diameter at the proximal end 50 compared to the distal end 52. See, e.g., Figure 36, discussed below. The tapered design may allow the prosthesis to conform better to the natural decreasing distal cross-section of the vessel, to reduce the risk of graft migration and potentially create better flow dynamics. The cage 46 can be provided with a proximal zone 55 and distal zone 59 that have a larger average expanded diameter than the central zone 57, as illustrated in Figure 2. This configuration may desirably resist migration of the prosthesis within the vessel and reduce leakage around the ends of the prosthesis.

The tubular wire support 46 may be formed from a continuous single length of round or flattened wire. Alternatively, two or more wire lengths can be secured together to produce the wire support 46. The wire support 46 is preferably formed in a plurality of discrete tubular segments 54, connected together and oriented about a common axis. Each pair of adjacent segments 54 is connected by a connector 66 as illustrated in Figure 3. The connectors 66 collectively produce a generally axially extending backbone which adds axial strength to the prosthesis 42. Adjacent segments can be connected both by the backbone, as well as the interlocking junction disclosed below. Additional structures, including circumferentially extending sutures, solder joints, and wire loops may also be used.

The segmented configuration of the tubular wire support 46 facilitates a great deal of flexibility. Each segment 54, though joined to adjacent segments, may be independently engineered to yield desired parameters. Each segment may range in axial length from about 0.3 to about 5 cm, and may be a uniform or non-uniform length around its circumference, such as to facilitate curvature as is discussed below. Generally, the shorter their length the greater the radial strength. An endoluminal prosthesis may include from about 1 to about 50 segments, preferably from about 3 to about 10 segments. For example, while a short graft patch, in accordance with the invention, may comprise only 2 segments and span a total of 2 to 3 cm, a complete graft may comprise 4 or more segments and span the entire aortic aneurysm. In addition to the flexibility and other functional benefits available through employment of different length segments, further flexibility can be achieved through adjustments in the number, angle, or configuration of the wire bends associated with the tubular support. See, e.g., Figures 34 and 35, discussed below.

In addition to having differing expanded diameters in different zones of the prosthesis 42 different zones can be provided with a different radial expansion force, such as ranging from about 0.89 N (.2 lbs.) to about 356 N (.8 lbs.). In one embodiment, the proximal zone 55 is provided with a greater radial force than the central zone 57 and/or distal zone 59. The greater radial force can be provided in any of a variety of manners discussed elsewhere herein, such as through the use of an additional one or two or three or more proximal bends 60, distal bends 62 and wall sections 64 compared to a reference segment 54 in the central zone 57 or distal zone 59. Alternatively, additional spring force can be achieved in the proximal zone 55 through the use of the same number of proximal bends 60 as in the rest of the prosthesis, but with a heavier gauge wire.

The wire may be made from any of a variety of different alloys, such as elgiloy, nitinol or MP35N, or other alloys which include nickel, titanium, tantalum, or stainless steel, high Co-Cr alloys or other temperature sensitive materials. For example, an alloy comprising Ni 15%, Co 40%, Cr 20%, Mo 7% and balance Fe may be used. The tensile strength of suitable wire is generally above about 2068 MPa (300 Ksi) and often between about 2068 and 2344 MPa (300 and about 340 Ksi) for many embodiments. In one embodiment, a Chromium-Nickel-Molybdenum alloy such as that marketed under the name Conichrom (Fort Wayne Metals, Indiana) has a tensile strength ranging from 2068 to 2206 MPa (300 to 320 K psi), elongation of 3.5 - 4.0%. The wire may be treated with a plasma coating and be provided with or without additional coatings such as PTFE, Teflon, Perlyne and drugs.

In addition to segment length and bend configuration, discussed above, another determinant of radial strength is wire gauge. The radial strength, measured at 50% of the collapsed profile, preferably ranges from about 0.89-3.56 N (0.2 lb. to 0.8 lb), and generally from about 1.779-2.224 N (0.4 lb. to about 0.5 lb.) or more. Preferred wire diameters in accordance with the present invention range from about 0.102 mm (0.004 inches) to about 0.508 mm (0.020 inches). More preferably, the wire diameters range from about 0.152 mm (0.006 inches) to about 0.457 mm (0.018 inches). In general, the greater the wire diameter, the greater the radial strength for a given wire layout. Thus, the wire gauge can be varied depending upon the application of the finished graft, in combination with/or separate from variation in other design parameters (such as the number of struts, or proximal bends 60 and distal bends 62 per segment), as will be discussed. A wire diameter of approximately 0.018 inches may be useful in a graft having four segments each having 2.5 cm length per segment, each segment having six struts intended for use in the aorta, while a smaller diameter such as 0.152 mm (0.006 inches) might be useful for a 0.5 cm segment graft having 5 struts per segment intended for the iliac artery. The length of cage 42 could be as long as about 28 cm.

In one embodiment of the present invention, the wire diameter is tapered from the proximal to distal ends. Alternatively, the wire diameter may be tapered incrementally or stepped down, or stepped up, depending on differing radial strength requirements along the length of the graft for each particular clinical application. In one embodiment, intended for the abdominal aortic artery, the wire has a cross-section of about 0.457 mm (0.018 inches) in the proximal zone 55 and the wire tapers down to a diameter of about 0.152 mm (0.006 inches) in the distal zone 59 of the graft 42. End point dimensions and rates of taper can be varied widely, within the spirit of the present invention, depending upon the desired clinical performance.

Referring to Figure 3, there is illustrated a plan view of a single formed wire used for rolling about a longitudinal axis to produce a four segment straight tubular wire support. The formed wire exhibits distinct segments, each corresponding to an individual tubular segment 54 in the tubular support (see Figures 1 and 2).

Each segment has a repeating pattern of proximal bends 60 connected to corresponding distal bends 62 by wall sections 64 which extend in a generally zig-zag configuration when the segment 54 is radially expanded. Each segment 54 is connected to the adjacent segment 54 through a connector 66, except at the terminal ends of the graft. The connector 66 in the illustrated embodiment comprises two wall or strut sections 64 which connect a proximal bend 60 on a first segment 54 with a distal bend 62 on a second, adjacent segment 54. The connector 66 may additionally be provided with a connector bend 68, which may be used to impart increased radial strength to the graft and/or provide a tie site for a circumferentially extending suture.

Referring to Figure 4, there is shown an enlarged view of the wire support illustrating a connector 66 portion between adjacent segments 54. In the embodiment shown in Figure 4, a proximal bend 60 comprises about a 180 degree arc, having a radial diameter of (w) (Ranging from 1.778-0.229 mm (.070 to .009 inches)), depending on wire diameter followed by a relatively short length of parallel wire spanning an axial distance of d1. The parallel wires thereafter diverge outwardly from one another and form the strut sections 64, or the proximal half of a connector 66. At the distal end of the strut sections 64, the wire forms a distal bend 62, preferably having identical characteristics as the proximal bend 60, except being concave in the opposite direction. The axial direction component of the distance between the apices of the corresponding proximal and distal bends 60, 62 on a given strut section 64 is referred to as (d) and represents the axial length of that segment. The total expanded angle defined by the bend 60 and the divergent strut sections 64 is represented by α. Upon compression to a collapsed state, such as when the graft is within the deployment catheter, the angle α is reduced to α'. In the expanded configuration, α is generally within the range of from about 35□ to about 450 for a six apex section having an axial length of about 1.5 cm or 2 cm and a diameter of about 25 mm or 28mm. The expanded circumferential distance between any two adjacent distal bends 62 (or proximal bends 60) is defined as (s).

In general, the diameter W of each proximal bend 60 or distal bend 62 is within the range of from about 0.229 mm (0.009 inches) to about 1.778 mm (0.070 inches) depending upon the wire diameter. Diameter W is preferably as small as possible for a given wire diameter and wire characteristics. As will be appreciated by those of skill in the art, as the distance W is reduced to approach two times the cross-section of the wire, the bend 60 or 62 will exceed the elastic limit of the wire, and radial strength of the finished segment will be lost. Determination of a minimum value for W, in the context of a particular wire diameter and wire material, can be readily determined through routine experimentation by those of skill in the art.

As will be appreciated from Fig. 3 and 4, the sum of the distances (s) in a plane transverse to the longitudinal axis of the finished graft will correspond to the circumference of the finished graft cage in that plane. For a given circumference, the number of proximal bends 60 or distal bends 62 is directly related to the distance (s) in the corresponding plane. Preferably, the finished graft in any single transverse plane will have from about 3 to about 10 (s) dimensions, preferably from about 4 to about 8 (s) dimensions and, more preferably, about 5 or 6 (s) dimensions for an aortic application. Each (s) dimension corresponds to the distance between any two adjacent bends 60-60 or 62-62 as will be apparent from the discussion herein. Each segment 54 can thus be visualized as a series of triangles extending circumferentially around the axis of the graft, defined by a proximal bend 60 and two distal bends 62 or the reverse.

In one embodiment of the type illustrated in Figure 4, w is about 2.0 mm [] 1 mm for a 0.457 mm (0.018 inch) wire diameter. D1 is about 3 mm [] 1 mm, and d is about 20 mm [] 1 mm. Specific dimensions for all of the foregoing variables can be varied considerably, depending upon the desired wire configuration, in view of the disclosure herein.

Referring to Figures 5 and 6, one or more apexes 76 is provided with an elongated axial length d2, which permits the apex 76 to be wrapped around a corresponding portion 78 such as an apex of the adjacent segment to provide an interlocking link 70 between two axially adjacent cage segments. In one embodiment of the link 70 produced by the opposing apexes 76 and 78 of Figure 6, utilizing wire having a diameter from 0.305 - 0.457 mm (.012" to .018"), d1 is generally within the range of from about 1 mm to about 4 mm and d2 is within the range of from about 5 mm to about 9 mm. In general, a longer d2 dimension permits accommodation for greater axial travel of apex 78 with respect to 76, as will be discussed, thereby permitting greater lateral flexibility of the graft. W1 is within the range of from about 3 mm to about 5 mm, and W2 is sufficiently less than W1 that the apex 76 can fit within the apex 78. Any of a wide variety of specific apex configurations and dimensions can be utilized, as will be apparent to those of skill in the art in view of the disclosure herein. Regardless of the specific dimensions, the end of the apex 76 is advanced through the apex 78, and folded back upon its self to hook the apex 78 therein to provide a link 70 in accordance with the present invention.

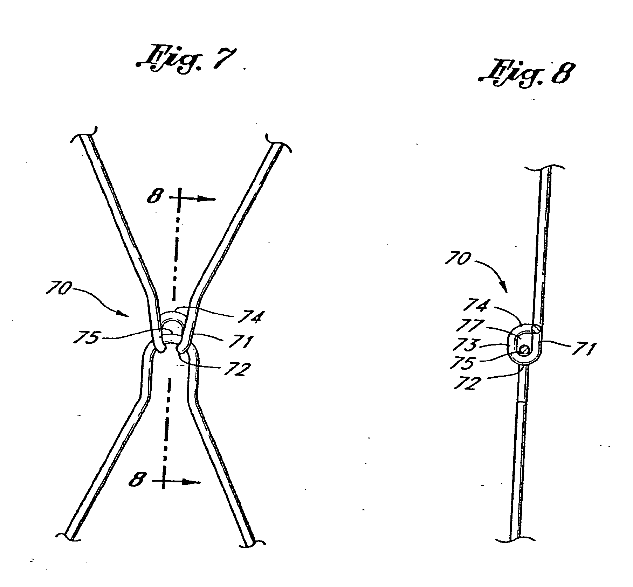

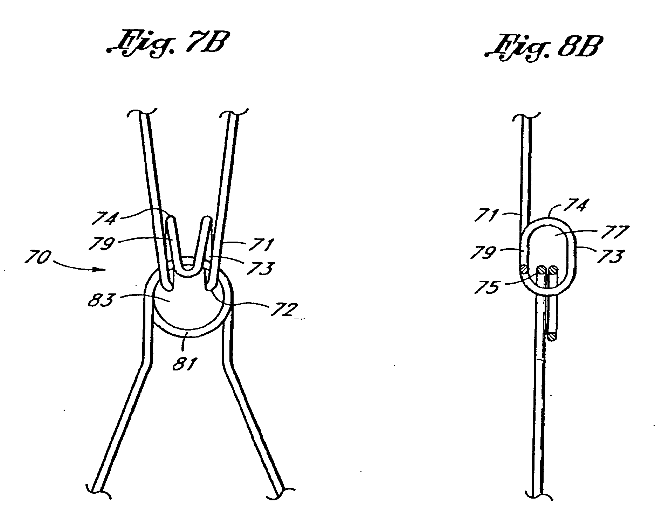

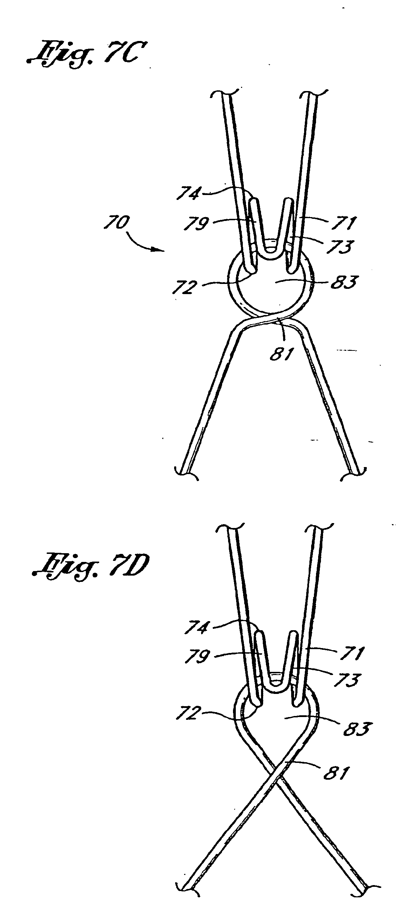

The resulting link 70 (see Figs. 7 and 8) comprises a wall portion 71 extending in a first direction, substantially parallel to the axis of the graft, and a transverse portion 72 extending transverse to the axis of the graft. A return portion 73 extends generally in the opposite direction from the wall portion 71 to create a generally "U" shaped hook. In certain embodiments, a closing portion 74 is also provided, to minimize the risk of excessive axial compression of the wire cage. The forgoing structure produces a functionally closed aperture 77, which receives the interlocking section 75 of the adjacent graft segment. Alternatively, see Figure 10.

In general, the aperture 77 preferably has a width (as viewed in Figure 8) in the radial graft direction of substantially equal to the radial direction dimension of the interlocking section 75. In this embodiment, the interlocking section 75, as well as the locking portion 71 and return portion 73 can be flattened in the radial direction, to minimize the transverse cross-section of the link 70. In the axial direction, the aperture 77 is preferably greater than the axial direction dimension of the interlocking section 75, to accommodate some axial movement of each adjoining tubular segment of the graft. The axial length of the aperture 77 is at least about 2 times, and preferably at least about 3 or 4 times the cross-section of the interlocking section 75. The optimum axial length of the aperture 77 can be determined through routine experimentation by one of skill in the art in view of the intended clinical performance, taking into account the number of links 70 per transverse plane as well as the desired curvature of the finished graft.

Figures 6A, 7A and 8A illustrate an alternate configuration for the moveable link 70. With this configuration, the radial expansion force will be higher.

Figures 7B and 8B illustrate another alternate configuration. This linkage has a better resistance to axial compression and disengagement. Referring to Figures 7B and 8B, the apex extends beyond closing portion 74 and into an axial portion 79 which extends generally parallel to the longitudinal axis of the graft. Provision of an axial extension 79 provides a more secure enclosure for the aperture 77 as will be apparent to those of skill in the art. The embodiments of Figure 7B and 8B also illustrate an enclosed aperture 83 on the opposing apex. The aperture 83 is formed by wrapping the apex in at least one complete revolution so that a generally circumferentially extending portion 81 is provided. Circumferential portion 81 provides a stop, to limit axial compressibility of the graft. The closed aperture 83 can be formed by winding the wire of the apex about a mandrel either in the direction illustrated in Figure 7B, or the direction illustrated in Figure 7C. The embodiment of Figure 7C advantageously provided only a single wire thickness through the aperture 77, thereby minimizing the wall thickness of the graft. This is accomplished by moving the crossover point outside of the aperture 77, as will be apparent from Figure 7C.

The link 70 in accordance with the present invention is preferably formed integrally with the wire which forms the cage of the endovascular prosthesis. Alternatively, link 70 may be constructed from a separate material -which is secured to the wire cage such as by soldering, suture, wrapping or the like.

The axial direction of the link 70 may also be varied, depending upon the desired performance characteristics of the graft. For example, the distal tips 76 of each link 70 may all face the same direction, such as proximal or distal with respect to the graft. See, for example, Figure 5. Alternatively, one or more links in a given transverse plane of apexes may face in a proximal direction, and one or more links in the same transverse plane may face in the opposite direction. See, for example, Figure 9.

Regardless of the axial orientation of the link 70, at least one and preferably at least two links 70 are provided per transverse plane separating adjacent graft segments. In an embodiment having six apexes per transverse plane, preferably at least two or three and in one embodiment all six opposing apex pairs are provided with a link 70. See Figure 5.

The distribution of the interlocking link 70 throughout the wire cage can thus vary widely, depending upon the desired performance characteristics. For example, each opposing apex pair between adjacent tubular segments can be provided with a link 70. See Figure 5. Alternatively, interlocking links 70 may be spaced circumferentially apart around the graft wall such as by positioning them at every second or third opposing apex pair.

The distribution of the links 70 may also be varied along the axial length of the graft. For example, a first zone at a proximal end of the graft and a second zone at a distal end of the graft may be provided with a relatively larger number of links 70 than a third zone in the central portion of the graft. In one embodiment, the transverse apex plane between the first and second tubular segments at the proximal end of the graft may be provided with a link 70 at each opposing apex pair. This has been determined by the present inventors to increase the radial strength of the graft, which may be desirable at the proximal (superior) end of the graft and possibly also at the distal end of the graft where resistance to leakage is an issue. A relatively lesser radial strength may be necessary in the central portion of the graft, where maintaining patency of the lumen is the primary concern. For this reason, relatively fewer links 70 may be utilized in a central zone, in an effort to simplify graft design as well as reduce collapse profile of the graft. See Figure 12.

In one straight segment graft, having four graft segments, three transverse apex planes are provided. In the proximal apex plane, each opposing pair of apexes is provided with a link 70. In the central transverse apex plane, three of the six apex pairs are provided with a links 70, spaced apart at approximately 120□. Substantially equal circumferential spacing of the link 70 is preferred, to provide relatively uniform resistance to bending regardless of graft position. The distal transverse apex plane may also be provided with a link 70 at each opposing apex pair.

The foregoing interlocking link 70 in accordance with the present invention can be readily adapted to both the straight segment grafts as discussed above, as well as to the bifurcated grafts discussed below.

The interlocking link 70 can be utilized to connect any of a number of independent graft segments in axial alignment to produce either a straight segment or a bifurcation graft. The interlocking link 70 may be utilized as the sole means of securing adjacent segments to each other, or may be supplemented by additional attachment structures such as metal loops, sutures, welds and others which are well understood in the art.

Referring to Figures 12A through 12C there is illustrated a further wire layout which allows a smaller collapsed profile for the vascular graft. In general, the embodiment of Figures 12A through 12C permits a series of links 70A and 70B to be staggered axially from one another as seen in Figure 12A and 12B. In this manner, adjacent links 70 do not lie in the same transverse plane, and permit a tighter nesting of the collapsed wire cage. Preferably, between each adjoining graft segment, at least a first group of links 70A is offset axially from a second group of links 70B. In a six apex graft, having a link 70 at each apex, for example, a first group of every other apex 70A may be positioned slightly proximally of a second group of every other apex 70B. Referring to Figure 12C, this may be accomplished by extending an apex 76A by a d3 distance which is at least about 1.2 times and as large as 1.5 times or 2 times or more the distance d2. The corresponding apexes 78 and 78A are similarly staggered axially, to produce the staggered interface between adjacent graft segments illustrated in Figure 12A. Although a loop apex is illustrated in Figure 12C as apex 78, any of the alternate apexes illustrated herein can be utilized in the staggered apex embodiment of the invention. The zig-zag pattern produced by axially offset links 70A and 70B can reside in a pair of parallel transverse planes extending generally between adjacent segments of the graft. Alternatively, the zig-zag relationship between adjacent links 70A and 70B can spiral around the circumference of a graft in a helical pattern, as will be understood by those of skill in the art in view of the disclosure herein. The precise axial offset between adjacent staggered links 70A and 70B can be optimized by one of ordinary skill in the art through routine experimentation, taking into account the desired physical properties and collapsed profile of the graft.

Referring to Figures 13 and 14, a straight segment deployment device and method in accordance with a preferred embodiment of the present invention are illustrated. A delivery catheter 80, having a dilator tip 82, is advanced along guidewire 84 until the (anatomically) proximal end 50 of the collapsed endoluminal vascular prosthesis 88 is positioned between the renal arteries 32 and 34 and the aneurysm 40. The collapsed prosthesis in accordance with the present invention has a diameter in the range of about 2 to about 10 mm. Generally, the diameter of the collapsed prosthesis is in the range of about 3 to 6 mm (12 to 18 French). Preferably, the delivery catheter including the prosthesis will be 16 F, or 15 F or 14 F or smaller.

The prosthesis 88 is maintained in its collapsed configuration by the restraining walls of the tubular delivery catheter 80, such that removal of this restraint would allow the prosthesis to self expand. Radiopaque marker material may be incorporated into the delivery catheter 80, and/or the prosthesis 88, at least at both the proximal and distal ends, to facilitate monitoring of prosthesis position. The dilator tip 82 is bonded to an internal catheter core 92, as illustrated in Figure 14, so that the internal catheter core 92 and the partially expanded prosthesis 88 are revealed as the outer sheath of the delivery catheter 80 is retracted.

As the outer sheath is retracted, the collapsed prosthesis 88 remains substantially fixed axially relative to the internal catheter core 92 and consequently, self-expands at a predetermined vascular site as illustrated in Figure 14. Continued retraction of the outer sheath results in complete deployment of the graft. After deployment, the expanded endoluminal vascular prosthesis 88 has radially self-expanded to a diameter anywhere in the range of about 20 to 40 mm, corresponding to expansion ratios of about 1:2 to 1:20. In a preferred embodiment, the expansion ratios range from about 1:4 to 1:8, more preferably from about 1:4 to 1:6.

In addition to, or in place of, the outer sheath described above, the prosthesis 88 may be maintained in its collapsed configuration by a restraining lace, which may be woven through the prosthesis or wrapped around the outside of the prosthesis in the collapsed reduced diameter. Following placement of the prosthesis at the treatment site, the lace can be proximally retracted from the prosthesis thereby releasing it to self expand at the treatment site. The lace may comprise any of a variety of materials, such as sutures, strips of PTFE, FEP, polyester fiber, and others as will be apparent to those of skill in the art in view of the disclosure herein. The restraining lace may extend proximally through a lumen in the delivery catheter or outside of the catheter to a proximal control. The control may be a pull tab or ring, rotatable reel, slider switch or other structure for permitting proximal retraction of the lace. The lace may extend continuously throughout the length of the catheter, or may be joined to another axially moveable element such as a pull wire.

In general, the expanded diameter of the graft in accordance with the present invention can be any diameter useful for the intended lumen or hollow organ in which the graft is to be deployed. For most arterial vascular applications, the expanded size will be within the range of from about 10 to about 40 mm. Abdominal aortic applications will generally require a graft having an expanded diameter within the range of from about 20 to about 28 mm, and, for example, a graft on the order of about 45 mm may be useful in the thoracic artery. The foregoing dimensions refer to the expanded size of the graft in an unconstrained configuration, such as on the table. In general, the graft will be positioned within an artery having a slightly smaller interior cross-section than the expanded size of the graft. This enables the graft to maintain a slight positive pressure against the wall of the artery, to assist in retention of the graft during the period of time prior to endothelialization of the polymeric sleeve 44.

The radial force exerted by the proximal segment 94 of the prosthesis against the walls of the aorta 30 provides a seal against the leakage of blood around the vascular prosthesis and tends to prevent axial migration of the deployed prosthesis. As discussed above, this radial force can be modified as required through manipulation of various design parameters, including the axial length of the segment and the bend configurations. In another embodiment of the present invention, radial tension can be enhanced at the proximal, upstream end by increasing the wire gauge in the proximal zone. Wire diameter may range from about 0.0254-0.254 mm (0.001 to 0.01 inches) in the distal region to a range of from about 0.254-0.762 mm (0.01 to 0.03 inches) in the proximal region.

An alternative embodiment of the wire layout which would cause the radial tension to progressively decrease from the proximal segments to the distal segments, involves a progressive or step-wise decrease in the wire gauge throughout the entire wire support, from about 0.254-0.762 mm (0.01 to 0.03 inches) at the proximal end to about 0.0508-0.254 mm (0.002 to 0.01 inches) at the distal end. Such an embodiment, may be used to create a tapered prosthesis. Alternatively, the wire gauge may be thicker at both the proximal and distal ends, in order to insure greater radial tension and thus, sealing capacity. Thus, for instance, the wire gauge in the proximal and distal segments may about 0.254-0.762 mm (0.01 to 0.03 inches), whereas the intervening segments may be constructed of thinner wire, in the range of about 0.0254-0.254 mm (0.001 to 0.01 inches).

Referring to Figure 15, there is illustrated two alternative deployment sites for the endoluminal vascular prosthesis 42 of the present invention. For example, an aneurysm 33 is illustrated in the right renal artery 32. An expanded endoluminal vascular prosthesis 42, in accordance with the present invention, is illustrated spanning that aneurysm 33. Similarly, an aneurysm 37 of the right common iliac 36 is shown, with a prosthesis 42 deployed to span the iliac aneurysm 37.

Referring to Figure 16, there is illustrated a modified embodiment of the endovascular prosthesis 96 in accordance with the present invention. In the embodiment illustrated in Figure 16, the endovascular prosthesis 96 is provided with a wire cage 46 having six axially aligned segments 54. As with the previous embodiments, however, the endovascular prosthesis 96 may be provided with anywhere from about 2 to about 10 or more axially spaced or adjacent segments 54, depending upon the clinical performance objectives of the particular embodiment.

The wire support 46 is provided with a tubular polymeric sleeve 44 as has been discussed. In the present embodiment, however, one or more lateral perfusion ports or openings are provided in the polymeric sleeve 44, such as a right renal artery perfusion port 98 and a left renal artery perfusion port 100 as illustrated.

Perfusion ports in the polymeric sleeve 44 may be desirable in embodiments of the endovascular prosthesis 96 in a variety of clinical contexts. For example, although Figures 1 and 16 illustrate a generally symmetrical aneurysm 40 positioned within a linear infrarenal portion of the abdominal aorta, spaced axially apart both from bilaterally symmetrical right and left renal arteries and bilaterally symmetrical right and left common iliacs, both the position and symmetry of the aneurysm 40 as well as the layout of the abdominal aortic architecture may differ significantly from patient to patient. As a consequence, the endovascular prosthesis 96 may need to extend across one or both of the renal arteries in order to adequately anchor the endovascular prosthesis 96 and/or span the aneurysm 40. The provision of one or more lateral perfusion ports or zones enables the endovascular prosthesis 96 to span the renal arteries while permitting perfusion therethrough, thereby preventing "stent jailing" of the renals. Lateral perfusion through the endovascular prosthesis 96 may also be provided, if desired, for a variety of other arteries including the second lumbar, testicular, inferior mesenteric, middle sacral, and alike as will be well understood to those of skill in the art.

The endovascular prosthesis 96 is preferably provided with at least one, and preferably two or more radiopaque markers, to facilitate proper positioning of the prosthesis 96 within the artery. In an embodiment having perfusion ports 98 and 100 such as in the illustrated design, the prosthesis 96 should be properly aligned both axially and rotationally, thereby requiring the ability to visualize both the axial and rotational position of the device. Alternatively, provided that the delivery catheter design exhibits sufficient torque transmission, the rotational orientation of the graft may be coordinated with an indexed marker on the proximal end of the catheter, so that the catheter may be rotated and determined by an external indicium of rotational orientation to be appropriately aligned with the right and left renal arteries.

In an alternative embodiment, the polymeric sleeve 44 extends across the aneurysm 40, but terminates in the infrarenal zone. In this embodiment, a proximal zone 55 on the prosthesis 96 comprises a wire cage 46 but no polymeric sleeve 44. In this embodiment, the prosthesis 96 still accomplishes the anchoring function across the renal arteries, yet does not materially interfere with renal perfusion. Thus, the polymeric sleeve 44 may cover anywhere from about 50% to about 100% of the axial length of the prosthesis 96 depending upon the desired length of uncovered wire cage 46 such as for anchoring and/or lateral perfusion purposes. In particular embodiments, the polymeric sleeve 44 may cover within the range of from about 70% to about 80%, and, in one four segment embodiment having a single exposed segment, 75%, of the overall length of the prosthesis 96. The uncovered wire cage 46 may reside at only a single end of the prosthesis 96, such as for traversing the renal arteries. Alternatively, exposed portions of the wire cage 46 may be provided at both ends of the prosthesis such as for anchoring purposes.

In a further alternative, a two part polymeric sleeve 44 is provided. A first distal part spans the aneurysm 40, and has a proximal end which terminates distally of the renal arteries. A second, proximal part of the polymeric sleeve 44 is carried by the proximal portion of the wire cage 46 which is positioned superiorly of the renal arteries. This leaves an annular lateral flow path through the side wall of the vascular prosthesis 96, which can be axially aligned with the renal arteries, without regard to rotational orientation.

The axial length of the gap between the proximal and distal segments of polymeric sleeve 44 can be adjusted, depending upon the anticipated cross-sectional size of the ostium of the renal artery, as well as the potential axial misalignment between the right and left renal arteries. Although the right renal artery 32 and left renal artery 34 are illustrated in Figure 16 as being concentrically disposed on opposite sides of the abdominal aorta, the take off point for the right or left renal arteries from the abdominal aorta may be spaced apart along the abdominal aorta as will be familiar to those of skill in the art. In general, the diameter of the ostium of the renal artery measured in the axial direction along the abdominal aorta falls within the range of from about 7 mm to about 20 mm for a typical adult patient.

Prior art procedures presently use a 7 mm introducer (18 French) which involves a surgical procedure for introduction of the graft delivery device. Embodiments of the present invention can be constructed having a outside diameter of 5.3 mm (16 French) or 5 mm (15 French) or 4.7 mm (14 French) or smaller profile (e.g. 3-4 mm) thereby enabling placement of the endoluminal vascular prosthesis of the present invention by way of a percutaneous procedure. In addition, the endoluminal vascular prosthesis of the present invention does not require a post implantation balloon dilatation, can be constructed to have minimal axial shrinkage upon radial expansion.

Referring to Figure 17, there is disclosed a schematic representation of the abdominal part of the aorta and its principal branches as in Figure 1. An expanded bifurcated endoluminal vascular prosthesis 102, in accordance with the present invention, is illustrated spanning the aneurysms 103,104 and 105. The endoluminal vascular prosthesis 102 includes a polymeric sleeve 106 and a tubular wire support 107, which are illustrated *in situ* in Figure 17. The sleeve 106 and wire support 107 are more readily visualized in the exploded view shown in Figure 19. The endoluminal prosthesis 102 illustrated and described herein depicts an embodiment in which the polymeric sleeve 106 is situated concentrically outside of the tubular wire support 107. However, other embodiments may include a sleeve situated instead concentrically inside the wire support or on both of the inside and the outside of the wire support. Alternatively, the wire support may be embedded within a polymeric matrix which makes up the sleeve. Regardless of whether the sleeve 106 is inside or outside the wire support 107, the sleeve may be attached to the wire support by any of a variety of means, as has been previously discussed.

The tubular wire support 107 comprises a primary component 108 for traversing the aorta and a first iliac, and a branch component 109 for extending into the second iliac. The primary component 108 may be formed from a continuous single length of wire, throughout both the aorta trunk portion and the iliac branch portion. See Figures 19 and 20. Alternatively, each iliac branch component can be formed separately from the aorta trunk portion. Construction of the graft from a three part cage conveniently facilitates the use of different gauge wire in the different components (e.g. 14 gauge main trunk and 10 gauge branch components).

The wire support 107 is preferably formed in a plurality of discrete segments, connected together and oriented about a common axis. In Figure 20, Section A corresponds to the aorta trunk portion of the primary component 108, and includes segments 1-5. Segments 6-8 (Section B) correspond to the iliac branch portion of the primary component 108.

In general, each of the components of the tubular wire support 107 can be varied considerably in diameter, length, and expansion coefficient, depending upon the intended application. For implantation within a typical adult, the aorta trunk portion (section A) of primary component 108 will have a length within the range of from about 5 cm to about 12 cm, and, typically within the range of from about 9 cm to about 10 cm. The unconstrained outside expanded diameter of the section A portion of the primary component 108 will typically be within the range of from about 20 mm to about 40 mm. The unconstrained expanded outside diameter of the section A portion of primary component 108 can be constant or substantially constant throughout the length of section A, or can be tapered from a relatively larger diameter at the proximal end to a relatively smaller diameter at the bifurcation. In general, the diameter of the distal end of section A will be on the order of no more than about 95% and, preferably, no more than about 85% of the diameter of the proximal end of section A.

The right and left iliac portions, corresponding to section B on primary component 108 and section C will typically be bilaterally symmetrical. Section C length will generally be within the range of from about 1 cm to about 5 cm, and section C diameter will typically be within the range of from about 10 mm to about 20 mm.

Referring to Figure 19, the wire cage 107 is dividable into a proximal zone 110, a central zone 111 and a distal zone 112. As has been discussed, the wire cage 107 can be configured to taper from a relatively larger diameter in the proximal zone 110 to a relatively smaller diameter in the distal zone 112. In addition, the wire cage 107 can have a transitional tapered and or stepped diameter within a given zone.

Referring to Figure 20, there is illustrated a plan view of the single formed wire used for rolling about a longitudinal axis to produce a primary segment 108 having a five segment aorta section and a three segment iliac section. The formed wire exhibits distinct segments, each corresponding to an individual tubular segment in the tubular support. Additional details of the wire cage layout and construction can be found in United States Patent No. 6,077,296 entitled Endoluminal Vascular Prosthesis, filed March 4, 1998.

Each segment has a repeating pattern of proximal bends 60 connected to corresponding distal bends 62 by wall sections 64 which extend in a generally zig-zag configuration when the segment is radially expanded, as has been discussed in connection with Figure 3. Each segment is connected to the adjacent segment through a connector 66, and one or more links 70 as has been discussed in connection with Figures 5-12. The connector 66 in the illustrated embodiment comprises two wall sections 64 which connect a proximal bend 60 on a first segment with a distal bend 62 on a second, adjacent segment The connector 66 may additionally be provided with a connector bend 68, which may be used to impart increased radial strength to the graft and/or provide a tie site for a circumferentially extending suture.

In the illustrated embodiment, section A is intended for deployment within the aorta whereas section B is intended to be deployed within a first iliac. Thus, section B will preferably have a smaller expanded diameter than section A. This may be accomplished by providing fewer proximal and distal bends 60, 62 per segment in section B or in other manners as will be apparent to those of skill in the art in view of the disclosure herein. In the illustrated embodiment, section B has one fewer proximal bend 60 per segment than does each segment in section A. This facilitates wrapping of the wire into a tubular prosthesis cage such as that illustrated in Figure 19, so that the iliac branch has a smaller diameter than the aorta branch. At the bifurcation, an opening remains for connection of the second iliac branch. The second branch is preferably formed from a section of wire in accordance with the general principles discussed above, and in a manner that produces a similarly dimensioned wire cage as that produced by section B. The second iliac branch (section C) may be attached at the bifurcation to section A and/or section B in any of a variety of manners, to provide a secure junction therebetween. In one embodiment, one or two of the proximal bends 60 on section C will be secured to the corresponding distal bends 62 on the distal most segment of section A. Attachment may be accomplished such as through the use of a circumferentially threaded suture, through links 70 as has been discussed previously, through soldering or other attachment means. The attachment means will be influenced by the desirable flexibility of the graft at the bifurcation, which will in turn be influenced by the method of deployment of the vascular graft as will be apparent to those of skill in the art in view of the disclosure herein.

Referring to Figure 21, there is disclosed an exploded schematic representation of a hinged or articulated variation in the tubular wire support structure for a bifurcated graft in accordance with present invention. The tubular wire support comprises a main body, or aortic trunk portion 200 and right 202 and left 204 iliac branch portions. Right and left designations correspond to the anatomic designations of right and left common iliac arteries. The proximal end 206 of the aortic trunk portion 200 has apexes 211-216 adapted for connection with the complementary apexes on the distal ends 208 and 210 of the right 202 and left 204 iliac branch portions, respectively. Complementary pairing of apexes is indicated by the shared numbers, wherein the right branch portion apexes are designated by (R) and the left branch portion apexes are designated by (L). Each of the portions may be formed from a continuous single length of wire. See Figure 23.

Referring to Figure 22, the assembled articulated wire support structure is shown. The central or medial -apex 213 in the foreground (anterior) of the aortic trunk portion 200 is linked with 213(R) on the right iliac portion 202 and 213(L) on the left iliac portion 204. Similarly, the central apex 214 in the background (posterior) is linked with 214(R) on the right iliac portion 202 and 214(L) on the left iliac portion 204. Each of these linkages has two iliac apexes joined with one aortic branch apex. The linkage configurations may be of any of the variety described above in Figure 7A-D. The medial most apexes 218 (R) and (L) of the iliac branch portions 202 and 204 are linked together, without direct connection with the aortic truck portion 200.

The medial apexes 213 and 214 function as pivot points about which the right and left iliac branches 202, 204 can pivot to accommodate unique anatomies. Although the right and left iliac branches 202, 204 are illustrated at an angle of about 45° to each other, they are articulable through at least an angle of about 90° and preferably at least about 120°. The illustrated embodiment allows articulation through about 180° while maintaining patency of the central lumen. To further improve patency at high iliac angles, the apexes 213 and 214 can be displaced proximally from the transverse plane which roughly contains apexes 211, 212, 215 and 216 by a minor adjustment to the fixture about which the wire is formed. Advancing the pivot point proximally relative to the lateral apexes (e.g., 211, 216) opens the unbiased angle between the iliac branches 202 and 204.

In the illustrated embodiment, the pivot point is formed by a moveable link between an eye on apex 213 and two apexes 213R and 213L folded therethrough. To accommodate the two iliac apexes 213R and 213L, the diameter of the eye at apex 213 may be slightly larger than the diameter of the eye on other apexes throughout the graft. Thus, for example, the diameter of the eye at apex 213 in one embodiment made from .014" diameter wire is about 0.059", compared to a diameter of about 0.020" for eyes elsewhere in the graft.

Although the pivot points (apexes 213, 214) in the illustrated embodiment are on the medial plane, they may be moved laterally such as, for example, to the axis of each of the iliac branches. In this variation, each iliac branch will have an anterior and a posterior pivot link on or about its longitudinal axis, for a total of four unique pivot links at the bifurcation. Alternatively, the pivot points can be moved as far as to lateral apexes 211 and 216. Other variations will be apparent to those of skill in the art in view of the disclosure herein.

To facilitate lateral rotation of the iliac branches 202, 204 about the pivot points and away from the longitudinal axis of the aorta trunk portion 200 of the graft, the remaining links between the aorta trunk portion 200 and the iliac branches 202, 204 preferably permit axial compression and expansion. In general, at least one and preferably several links lateral to the pivot point in the illustrated embodiment permit axial compression or shortening of the graft to accommodate lateral pivoting of the iliac branch. If the pivot point is moved laterally from the longitudinal axis of the aorta portion of the graft, any links medial of the pivot point preferably permit axial elongation to accommodate lateral rotation of the branch. In this manner, the desired range of rotation of the iliac branches may be accomplished with minimal deformation of the wire, and with patency of the graft optimized throughout the angular range of motion.

To permit axial compression substantially without deformation of the wire, the lateral linkages, 211 and 212 for the right iliac, and 215 and 216 for the left iliac, may be different from the previously described apex-to-apex linkage configurations. The lateral linkages are preferably slidable linkages, wherein a loop formed at the distal end of the iliac apex slidably engages a strut of the corresponding aortic truck portion. The loop and strut orientation may be reversed, as will be apparent to those of skill in the art. Interlocking "elbows" without any distinct loop may also be used. Such an axially compressible linkage on the lateral margins of the assembled wire support structure allow the iliac branch portions much greater lateral flexibility, thereby facilitating placement in patients who often exhibit a variety of iliac branch asymmetries and different angles of divergence from the aortic trunk.

Referring to Figure 23, there is illustrated a plan view of a single formed wire used for rolling about a longitudinal axis to produce a four segment straight tubular wire support for an iliac limb. The formed wire exhibits distinct segments, each corresponding to an individual tubular segment in the tubular supports 202 or 204 (See Figure 21). The distal segment I, is adapted to articulate with the aortic trunk portion 200 and the adjacent iliac limb portion. The distal segment (I) has two apexes (e.g. corresponding to 211 and 212 on the right iliac portion 202 in Figure 21) which form a loop adapted to slidably engage a strut in the lateral wall of the aortic portion. These articulating loops (A) are enlarged in Figure 24A. As discussed above, the loops are preferably looped around a strut on the corresponding apex of the proximal aortic segment to provide a sliding linkage.

The apex 218 is proximally displaced relative to the other four apexes in the distal segment (I). Apex 218 (R or L) is designed to link with the complementary 218 apex on the other iliac branch portion (See Figure 22). The apex 218 in the illustrated embodiment is formed adjacent or near an intersegment connector 66, which extends proximally from the distal segment.

The other apexes on the distal segment (I) of an iliac limb are designed to link with a loop on the corresponding apex of the proximal aortic segment. Because many variations of this linkage are consistent with the present invention (See Figures 7A-D), the form of the corresponding apexes may vary. In a preferred variation, the apexes (B) form a narrow U-shape, having an inside diameter of about 0.482 mm (0.019 inches) in an embodiment made from 0.3048 mm (0.012 inch) Conichrome wire (tensile strength 2068 MPa (300 ksi) minimum) as illustrated in Figure 24B. The U-shaped, elongated axial portion of the apex shown in Figure 24B permits the apex to be wrapped through and around a corresponding loop apex of the proximal aortic segment. This type of linkage is discussed in greater detail above in connection with Figures 5 and 6.

In more general terms, the wire support illustrated in Figures 21 and 22 comprises a main body support structure formed from one or more lengths of wire and having a proximal end, a distal end and a central lumen extending along a longitudinal axis. The wire support also comprises a first branch support structure formed from one or more lengths of wire and having a proximal end, a distal end and a central lumen therethrough. The first branch support structure is pivotably connected to the proximal end of the main body support structure. The tubular wire support further comprises a second branch support structure formed from one or more lengths of wire and having a proximal end, a distal end and a central lumen extending therethrough. The distal end of the second branch support structure is pivotably connected to the proximal end of the main body support structure.

Further, the distal ends of the first and second branch structures may be joined together by a flexible linkage, formed for example between apexes 218(R) and 218(L) in Figure 21. By incorporating a medial linkage between the two branch support structures and pivotable linkages with the main trunk, the first and second branch support structures can hinge laterally outward from the longitudinal axis without compromising the volume of the lumen. Thus, the branches may enjoy a wide range of lateral movement, thereby accommodating a variety of patient and vessel heterogeneity. Additional corresponding apexes between the main trunk and each iliac branch may also be connected, or may be free floating within the outer polymeric sleeve. Axially compressible lateral linkages, discussed above and illustrated in Figure 22, may optionally be added.

The proximal apexes (C) of the iliac limb portions are adapted to link with the distal apexes of the next segment These proximal apexes preferably form loops, such as those illustrated in Figure 24C, wherein the elongated axial portions of the corresponding proximal apex in the adjacent segment can wrap around the loop, thereby providing flexibility of the graft, as discussed above for Figures 5 and 6.

The wire may be made from any of a variety of different alloys and wire diameters or non-round cross-sections, as has been discussed. In one embodiment of the bifurcation graft, the wire gauge remains substantially constant throughout section A of the primary component 49 and steps down to a second, smaller cross-section throughout section B of primary component 108.

A wire diameter of approximately 0.4572 mm (0.018 inches) may be useful in the aorta trunk portion of a graft having five segments each having 2.0 cm length per segment, each segment having six struts intended for use in the aorta, while a smaller diameter such as 0.3048 mm (0.012 inches) might be useful for segments of the graft having 6 struts per segment intended for the iliac artery.

In one embodiment of the present invention, the wire diameter may be tapered throughout from the proximal to distal ends of the section A and/or section B portions of the primary component 108. Alternatively, the wire diameter may be tapered incremental or stepped down, or stepped up, depending on the radial strength requirements of each particular clinical application. In one embodiment, intended for the abdominal aortic artery, the wire has a cross-section of about 0.4572 mm (0.018 inches) in the proximal zone 110 and the wire tapers down regularly or in one or more steps to a diameter of about 0.3048 mm (0.012 inches) in the distal zone 112 of the graft 102. End point dimensions and rates of taper can be varied widely, within the spirit of the present invention, depending upon the desired clinical performance.

In general, in the tapered or stepped wire embodiments, the diameter of the wire in the iliac branches is no more than about 80% of the diameter of the wire in the aortic trunk. This permits increased flexibility of the graft in the region of the iliac branches, which has been determined by the present inventors to be clinically desirable.

The collapsed prosthesis in accordance with the present invention has a diameter in the range of about 2 to about 10 mm. Preferably, the maximum diameter of the collapsed prosthesis is in the range of about 3 to 6 mm (12 to 18 French). Some embodiments of the delivery catheter including the prosthesis will be in the range of from 6 - 7 mm (18 to 20 or 21 French); other embodiments will be as low as 6.3 - 4.7 mm (19 F, 16 F, 14 F), or smaller. After deployment, the expanded endoluminal vascular prosthesis has radially self-expanded to a diameter anywhere in the range of about 20 to 40 mm, corresponding to expansion ratios of about 1:2 to 1:20. In a preferred embodiment, the expansion ratios range from about 1:4 to 1:8, more preferably from about 1:4 to 1:6.

The self expandable bifurcation graft of the present invention can be deployed at a treatment site in accordance with any of a variety of techniques as will be apparent to those of skill in the art. One such technique is disclosed in copending patent application serial No. 08/802,478 entitled Bifurcated Vascular Graft and Method and Apparatus for Deploying Same, filed February 20, 1997.

A partial cross-sectional side elevational view of one deployment apparatus 120 which can be used with the present invention is shown in Figure 25. The deployment apparatus 120 comprises an elongate flexible multicomponent tubular body 122 having a proximal end 124 and a distal end 126. The tubular body 122 and other components of this system can be manufactured in accordance with any of a variety of techniques well known in the catheter manufacturing field. Suitable materials and dimensions can be readily selected taking into account the natural anatomical dimensions in the iliacs and aorta, together with the dimensions of the desired percutaneous access site.

The elongate flexible tubular body 122 comprises an outer sheath 128 which is axially movably positioned upon an intermediate tube 130. A central tubular core 132 is axially movably positioned within the intermediate tube 130. In one embodiment, the outer tubular sheath comprises extruded PTFE, having an outside diameter of about 6.35 mm (.250") and an inside diameter of about 5.84 mm (.230"). The tubular sheath 128 is provided at its proximal end with a manifold 134, having a hemostatic valve 136 thereon and access ports such as for the infusion of drugs or contrast media as will be understood by those of skill in the art.

The outer tubular sheath 128 has an axial length within the range of from about 1.016 m (40") to about 1.397 m (55"), and, in one embodiment of the deployment device 120 having an overall length of 110 cm, the axial length of the outer tubular sheath 128 is about 52 cm and the outside diameter is no more than about 6.35 mm (.250"). Thus, the distal end of the tubular sheath 128 is located at least about 16 cm proximally of the distal end 126 of the deployment catheter 120 in stent loaded configuration.

As can be seen from Figures 26 and 27-28, proximal retraction of the outer sheath 128 with respect to the intermediate tube 130 will expose the compressed iliac branches of the graft, as will be discussed in more detail below.

A distal segment of the deployment catheter 120 comprises an outer tubular housing 138, which terminates distally in an elongate flexible tapered distal tip 140. The distal housing 138 and tip 140 are axially immovably connected to the central core 132 at a connection 142.

The distal tip 140 preferably tapers from an outside diameter of about 5.715 mm (.225") at its proximal end to an outside diameter of about 1.778 mm (.070") at the distal end thereof. The overall length of the distal tip 140 in one embodiment of the deployment catheter 120 is about 76.2 mm (3"). However, the length and rate of taper of the distal tip 140 can be varied depending upon the desired trackability and flexibility characteristics. The distal end of the housing 138 is secured to the proximal end of the distal tip 140 such as by thermal bonding, adhesive bonding, and/or any of a variety of other securing techniques known in the art. The proximal end of distal tip 140 is preferably also directly or indirectly connected to the central core 132 such as by a friction fit and/or adhesive bonding.

In at least the distal section of the catheter, the central core 132 preferably comprises a length of hypodermic needle tubing. The hypodermic needle tubing may extend throughout the length catheter to the proximal end thereof, or may be secured to the distal end of a proximal extrusion as illustrated for example in Figure 22. A central guidewire lumen 144 extends throughout the length of the tubular central core 132, having a distal exit port 146 and a proximal access port 148 as will be understood by those of skill in the art.

Referring to Figures 26-28, a bifurcated endoluminal graft 150 is illustrated in a compressed configuration within the deployment catheter 120. The graft 150 comprises a distal aortic section 152, a proximal ipsilateral iliac portion 154, and a proximal contralateral iliac portion 156. The aortic trunk portion 152 of the graft 150 is contained within the tubular housing 138. Distal axial advancement of the central tubular core 132 will cause the distal tip 140 and housing 138 to advance distally with respect to the graft 150, thereby permitting the aortic trunk portion 152 of the graft 150 to expand to its larger, unconstrained diameter. Distal travel of the graft 150 is prevented by a distal stop 158 which is axially immovably connected to the intermediate tube 130. Distal stop 158 may comprise any of a variety of structures, such as an annular flange or component which is adhered to, bonded to or integrally formed with a tubular extension 160 of the intermediate tube 132. Tubular extension 160 is axially movably positioned over the hypotube central core 132.

The tubular extension 160 extends axially throughout the length of the graft 150. At the proximal end of the graft 150, a step 159 axially immovably connects the tubular extension 160 to the intermediate tube 130. In addition, the step 159 provides a proximal stop surface to prevent proximal travel of the graft 150 on the catheter 120. The function of step 159 can be accomplished through any of a variety of structures as will be apparent to those of skill in the art in view of the disclosure herein. For example, the step 159 may comprise an annular ring or spacer which receives the tubular extension 160 at a central aperture therethrough, and fits within the distal end of the intermediate tube 130. Alternatively, the intermediate tube 130 can be reduced in diameter through a generally conical section or shoulder to the diameter of tubular extension 160.

Proximal retraction of the outer sheath 128 will release the iliac branches 154 and 156 of the graft 150. The iliac branches 154 and 156 will remain compressed, within a first (ipsilateral) tubular sheath 162 and a second (contralateral) tubular sheath 164. The first tubular sheath 162 is configured to restrain the ipsilateral branch of the graft 150 in the constrained configuration, for implantation at the treatment site. The first tubular sheath 162 is adapted to be axially proximally removed from the iliac branch, thereby permitting the branch to expand to its implanted configuration. In one embodiment, the first tubular sheath 162 comprises a thin walled PTFE extrusion having an outside diameter of about 5.461 mm (.215") and an axial length of about 7.5 cm. A proximal end of the tubular sheath 162 is necked down such as by heat shrinking to secure the first tubular sheath 162 to the tubular extension 160. In this manner, proximal withdrawal of the intermediate tube 130 will in turn proximally advance the first tubular sheath 162 relative to the graft 150, thereby deploying the self expandable iliac branch of the graft 150.

The second tubular sheath 164 is secured to the contralateral guidewire 166, which extends outside of the tubular body 122 at a point 168, such as may be conveniently provided at the junction between the outer tubular sheath 128 and the distal housing 138. The second tubular sheath 164 is adapted to restrain the contralateral branch of the graft 150 in the reduced profile. In one embodiment of the invention, the second tubular sheath 164 has an outside diameter of about 5.461 mm (.215") and an axial length of about 7.5 cm. The second tubular sheath 164 can have a significantly smaller cross-section than the first tubular sheath 162, due to the presence of the tubular core 132 and intermediate tube 130 within the first iliac branch 154.

The second tubular sheath 164 is secured at its proximal end to a distal end of the contralateral guidewire 166. This may be accomplished through any of a variety of securing techniques, such as heat shrinking, adhesives, mechanical interfit and the like. In one embodiment, the guidewire is provided with a knot or other diameter enlarging structure to provide an interference fit with the proximal end of the second tubular sheath 156, and the proximal end of the second tubular sheath 156 is heat shrunk and/or bonded in the area of the knot to provide a secure connection. Any of a variety of other techniques for providing a secure connection between the contralateral guidewire 166 and tubular sheath 156 can readily be used in the context of the present invention as will be apparent to those of skill in the art in view of the disclosure herein. The contralateral guidewire 166 can comprise any of a variety of structures, including polymeric monofilament materials, braided or woven materials, metal ribbon or wire, or conventional guidewires as are well known in the art.

In use, the free end of the contralateral guidewire 166 is percutaneously inserted into the arterial system, such as at a first puncture in a femoral artery. The contralateral guidewire is advanced through the corresponding iliac towards the aorta, and crossed over into the contralateral iliac in accordance with cross over techniques which are well known in the art. The contralateral guidewire is then advanced distally down the contralateral iliac where it exits the body at a second percutaneous puncture site.

The deployment catheter 120 is thereafter percutaneously inserted into the first puncture, and advanced along a guidewire (e.g. 0.889 mm (0.035 inch) through the ipsilateral iliac and into the aorta. As the deployment catheter 120 is transluminally advanced, slack produced in the contralateral guidewire 166 is taken up by proximally withdrawing the guidewire 166 from the second percutaneous access site. In this manner, the deployment catheter 120 is positioned in the manner generally illustrated in Figure 29. Referring to Figure 30, the outer sheath 128 is proximally withdrawn while maintaining the axial position of the overall deployment catheter 120, thereby releasing the first and second iliac branches of the graft 150. Proximal advancement of the deployment catheter 120 and contralateral guidewire 166 can then be accomplished, to position the iliac branches of the graft 150 within the iliac arteries as illustrated.

Referring to Figure 31, the central core 132 is distally advanced thereby distally advancing the distal housing 138 as has been discussed. This exposes the aortic trunk of the graft 150, which deploys into its fully expanded configuration within the aorta. As illustrated in Figure 32, the contralateral guidewire 166 is thereafter proximally withdrawn, thereby by proximally withdrawing the second sheath 164 from the contralateral iliac branch 156 of the graft 150. The contralateral branch 156 of the graft 150 thereafter self expands to fit within the iliac artery. The guidewire 166 and sheath 164 may thereafter be proximally withdrawn and removed from the patient, by way of the second percutaneous access site.

Thereafter, the deployment catheter 120 may be proximally withdrawn to release the ipsilateral branch 154 of the graft 150 from the first tubular sheath 162 as shown in Figure 33. Following deployment of the ipsilateral branch 154 of the prosthesis 150, a central lumen through the aortic trunk 152 and ipsilateral branch 154 is sufficiently large to permit proximal retraction of the deployment catheter 120 through the deployed bifurcated graft 150. The deployment catheter 120 may thereafter be proximally withdrawn from the patient by way of the first percutaneous access site.

Referring to Figures 34a-34c, there is illustrated a flexible, straight graft design in accordance with the present invention. In this configuration, the wire cage may be flexed around a bend by allowing axial compression on an inside radius 227 compared to an outside radius 229, as seen in Figure 34c. Axial compression on the inside radius 227 is enabled by the provision of a plurality of sliding links 220, one embodiment of which is illustrated in Figure 34b. In general, the sliding link 220 enables the connection between a proximal bend 60 and a corresponding distal bend 62 to be compressible in the axial direction while maintaining the radial strength and radial expandability of the graft.

In the illustrated embodiment, this is accomplished by looping the proximal bend 60 around a wall section 64 which extends between a distal bend 62 and proximal bend 60 on a proximally adjacent segment of the graft. The proximal bend 60 is formed into a closed loop to form an aperture 61 for slidably entrapping the corresponding wall section 64. As used herein, the designations "proximal" and "distal" may be interchangeable when used, for example, to describe the relative position of the complementary sliding link structures on a graft. In addition, the proximal and distal designations are used sometimes herein with respect to the deployment catheter or deployment catheter direction, and other times in their anatomical sense with respect to the heart. The particular usage of these terms will be apparent to those of skill in the art in the context in which they are used herein.

Thus, referring to Figure 34a, there is disclosed a flexible straight graft having a proximal end 50, and a distal end 52. The graft comprises a plurality of segments, such as a first segment 230, a second segment 232, a third segment 234, and a fourth segment 236. The adjacent segments can be formed separately or formed integrally such as from a single length of wire. All of the dimensions, materials and other design specifications which have been disclosed previously herein for both straight and bifurcated grafts may be provided with the sliding link design, and will therefore not be repeated. In general, however, the sliding link 220 is preferably provided on each of the connections between any two adjacent segments in a graft at which flexibility is desired, to permit the desired flexibility of the graft. For example, each of the connections between first segment 230 and second segment 232 may be provided with a sliding link 220. The connections between the intermediate segments 232 and 234 may be provided with either sliding links 220 or nonsliding links as have been disclosed elsewhere herein. The connections between the third segment 234 and fourth segment 236 may be provided with sliding links 220.

As illustrated in Figure 34b, the sliding link 220 may slide along the wall section 64 all the way to the proximal bend 60. This range of motion may permit an undesirable degree of graft shortening or other disadvantages, and may not be necessary to achieve the radius of curvature likely to be encountered in the normal range of anatomical variation. Referring to Figure 34d, the axial travel of the sliding link 220 along the wall section 64 may be limited by the provision of a limit or lock 221. The lock 221 in the illustrated embodiment comprises a loop 223 of the wire from which wall section 64 is formed. The lock 221 may be.provided anywhere between the distal bend 62 and the proximal bend 60, along the length of the wall section 64, depending upon the desired range of travel. The length of the wall section 64 in between the distal bend 62 and the lock 221 provides an axial compression range for the associated sliding link 220. The permitted range of travel for sliding link 220 may be varied depending upon the location of the sliding link 220 on the graft, and depending upon the desired radius of curvature for the graft in the implanted orientation.

The lock 221 may be formed as a single or double loop of the wire 64. Alternatively, the lock 221 may be separately formed and attached to the wall section 64 such as by soldering, brazing, adhesives, or by tying one or more knots in a suture or other material. A sliding link lock 221 may be provided for each sliding link 220 in a graft, or for only select sliding links 220, depending upon the desired clinical performance.

Referring to Figures 35a-35d, there is illustrated an embodiment of a bifurcation graft of the present invention in which sliding links 220 are utilized to increase the lateral flexibility of the proximal and distal ends of the graft, while axially noncompressible links are utilized for the central trunk of the graft. Referring to Figure 35a, the bifurcation graft extends between a proximal end 50 and a distal end 52. In a proximal zone 55, which may comprise two or three or more adjacent segments, the connections between each proximal bend 60 and corresponding distal bend 62 is in the form of a sliding link 220. See Figure 35b.

Throughout an intermediate zone 57, which may comprise the third, fourth, and fifth segments of the aortic trunk portion of the graft, axially noncompressible links are provided such as those disclosed elsewhere herein. See Figure 35c.

The distal zone 59 of the graft, which, in the context of a bifurcation graft, includes a portion or all of the right and left iliac branches, comprises sliding links 220. See Figure 35d. This assembly allows the maintenance of a certain axial (column strength) integrity while permitting the proximal end 50 and distal end 52 to adapt to curved or otherwise unusual anatomy. Sliding links 220 may alternatively be used throughout the length of the graft, or only at the connection of the proximal-most segment and/or the distal-most segment of the graft.

Referring to Figure 36, there is illustrated a tapered graft design. This design is illustrated in the context of a bifurcation graft, although it can be readily adapted for use on a straight segment graft such as that illustrated in Figure 1. It may be readily applied to any of the bifurcation grafts disclosed herein, including, for example, those of Figures 17-22.

In the illustrated embodiment, an aortic trunk 222 is connected to or formed with a right iliac branch 224 and a left iliac branch 226. At least a portion of the aortic trunk 222 is tapered from a larger unconstrained expanded diameter at the proximal end 50 to a smaller unconstrained expanded diameter at the bifurcation into right iliac 224 and left iliac 226. Although Figure 36 illustrates a relatively uniform taper throughout the length of the aortic trunk portion 222, a nonuniform taper may also be utilized. For example, the first segment 230 and second segment 232 may be provided with a taper while the third segment 234, fourth segment, and fifth segment 238 may be relatively nontapered. Any of a wide variety of alternate configurations may be devised, depending upon the desired clinical performance and intended anatomy.

In the illustrated embodiment, the first segment 230 is provided with 10 proximal bends 60. The fifth segment 238 is provided with six proximal bends 60. By successively reducing the number of proximal bends 60 (and thus the number of zig-zag components to each segment), the tapered design can be achieved. The reduction in proximal bends 60, and thus diameter of the aortic trunk portion 222, from the first segment 230 to the fifth or other last segment 238 may be such that the number of proximal bends 60 in the last segment 238 is anywhere from about 40% to about 100% of the number of proximal bends 60 in the first segment 230. In some embodiments, the last segment 238 has anywhere from about 50% to about 80% and, in certain embodiments, about 60% of the number of proximal bends 60 in the first segment 230.

The foregoing structure may be provided in any of a variety of expanded diameters. In general, for an abdominal aortic aneurysm at the bifurcation of the iliacs, the maximum expanded diameter of the first segment 230 is preferably at least about 25 mm, and in many embodiments, at least about 30 mm. In one embodiment, the expanded diameter of the first segment 230 is at least about 34 mm. The expanded diameter of the graft at the bifurcation is generally within the range of from about 22 mm to about 28 mm, and, in one embodiment, is no more than about 25 mm.

In an alternate embodiment, the tapered expanded diameter configuration may be achieved by shaping the PTFE graft into a tapered configuration which constrains expansion of the wire cage. In this embodiment, each segment of the wire cage may have the same number of proximal bends 60. However, the constraint imposed by the PTFE sleeve may produce an unnecessary and potentially undesirable bunching of the zig-zag portions of the wire frame, particularly in the area of the last segment 238.

Referring to Figure 37, there is illustrated a graft 250 which includes a feature which may be added onto any of the embodiments disclosed previously herein, to facilitate curvature of the graft. The precurved graft 250 may be straightened for mounting on the deployment catheter, but is configured in a manner that facilitates curvature as deployed.

The precurved graft 250 includes a plurality of segments 256 as has been discussed previously. Each segment 256 comprises a plurality of proximal bends 60 and distal bends 62 separated by wall sections 64. In previous embodiments, the axial length of the wall sections 64 within a given segment 256 have been generally the same. In the embodiment of Figure 37, however, at least one wall section 64b in a given segment 256 has a shorter length than at least one wall section 64a on the same segment 256. In a graft segment 256 having, for example,12 wall sections 64, generally from about 2 to about 6 wall sections 64b will have a shorter length than the remainder of the wall sections 64 in that segment 256.

Upon deployment, the wall sections 64b having the shorter length will limit elongation of the graft. The opposing wall sections 64a having a longer length will allow a plurality of sliding links to extend the length of the graft thereby enabling a curve, as illustrated, having a concave side 252 and a convex side 254.

In the illustrated embodiment, the overall curvature of the graft 250 is approximately 90°. Utilizing the differential length segments 256 of the present invention, grafts or graft sections may range from linear, to curved as much as 90° or 120° or more, including as much as 180° such as for use in exotic anatomies. This may be accomplished by providing sliding links on either or both of the convex side 254 and concave side 252 of the graft 250. In one embodiment, sliding links are provided on the convex side 254 and axially fixed links are provided on the concave side 252. This minimizes intrusion of the wire cage into the central lumen on the concave side 252 of the graft 250, which may otherwise occur upon axial compression of the graft on the concave side 252.

In accordance with a further feature of the present invention, a variety of modifications may be added to any of the grafts disclosed previously herein to increase resistance to migration and/or endoleaks, such as at the anatomically proximal end of the implanted graft. Thus, referring to Figure 38a, a graft 42 is illustrated having a plurality of proximal bends 60 on the anatomically proximal end (distal device end) of the graft 42. One or more of the proximal bends 60 is provided with a radially outwardly inclined barb 260 for increasing resistance to migration of the graft 42 within the vessel. In an embodiment having eight proximal bends 60, preferably at least two, and in some embodiments as many as four or six or all eight of the proximal bends 60 is provided with a barb 260. Generally, at least about 50% of the proximal bends 60 or associated struts will be provided with a barb or tread 260.

As illustrated, each barb 260 is integrally formed with the support structure of the graft 42, such as by forming the barb 260 as a part of the wire cage. This eliminates the need for a separately attached component, which may become disengaged and/or increases the complexity of the manufacturing process.

Referring to Figure 38b, the barb 260 may be formed by bending the proximal bend 60 radially outwardly at a bend point 262. The radial outwardly most tip of the barb 260 may be formed with or without a loop or eye as has been discussed. The bend 262 may be formed to cause the barb 260 to extend outwardly at an angle with respect to the longitudinal axis of the graft. The angle may range, in an unconstrained form, from anywhere within the range of from about 10° to about 160° from the longitudinal axis. In the illustrated embodiment, the bend 262 produces an angle of approximately 90°.

Although not illustrated in Figure 38a, the barbs 260 may be offset from each other or staggered with respect to the longitudinal axis of the graft 42, to minimize the collapsed profile of the graft 42 when loaded on the deployment catheter. Staggering of the barbs 260 may be accomplished by positioning the barbs 260 such that no two barbs 260 are axially aligned in a single transverse plane upon collapse of the graft 42. Alternatively, the barbs 260 may be alternated between a first transverse plane and a second transverse plane or a first, a second, and a third transverse plane as will be apparent to those of skill in the art in view of the disclosure herein. A similar staggering may be accomplished with respect to the plurality of links 258 in-between any adjacent segments in the graft, in any of the embodiments disclosed herein. This is illustrated in Figure 38a in a stagger pattern such that about half of the links 258 in a given joint between adjacent graft segments reside in a first transverse plane and the other half reside in a second transverse plane. This permits a tighter nesting or lower crossing profile for the collapsed graft. This feature is independent of the barbs 260, and either or both feature may be incorporated into any of the preceding embodiments.

A modified barb 260 is illustrated in Figure 38c. In this embodiment, a portion of the wall sections 264 are bent in a first, radially inwardly inclined direction and then provided with a second bend to direct the tip of the barb 260 in a radially outwardly inclined direction. Other barb configurations can be readily achieved, as will be apparent to those of skill in the art in view of the disclosure herein.

A further feature of the present invention which may be provided on any of the embodiments discussed previously herein is illustrated in Figures 39a-c. In accordance with this feature, an exoskeleton 264 is provided at some point on the radially outwardly facing surface of the graft fabric, to facilitate endothelialization or other biological encorporation mechanism for providing resistance to migration of the implanted graft. The exoskeleton 264 is particularly useful in an embodiment wherein the fabric of the graft resists or does not permit tissue ingrowth.

Referring to Figure 39a, each proximal bend 60 at the anatomically proximal end of the graft is connected by a generally V- or U-shaped partial exoskeleton segment 266. The exoskeleton segment 266 may extend beyond the end of the fabric, or may be folded back on top of the fabric as illustrated in Figure 39a. Each exoskeleton segment 266 terminates proximally in a bend 268 which may or may not include a loop or eye as has been discussed. The axial distance along the graft between the proximal bend 60 and adjacent exoskeleton bend 268 may be varied, depending upon the desired clinical performance. Generally, that axial distance is within the range of from about 1.5 cm to about 2.5 cm.

In addition, the plurality of bends 268 may be offset in the axial direction from a common transverse plane, to reduce the collapsed crossing profile of the graft as has been discussed. One or two or more of the bends 268 may be inclined radially outwardly and/or biased radially outwardly to resist migration.

A partial exoskeleton 264 may also be accomplished by leaving one or more adjacent apexes between adjacent graft segments unconnected from each other, and sliding the fabric of the graft beneath the unattached graft apex. This is illustrated, for example, in Figure 39b. In the illustrated embodiment, alternating distal bends 62 from the anatomically proximal-most graft segment remain on the outside of the graft fabric, while the remaining half of the distal bends 62 of the proximal-most graft segment remain connected to corresponding proximal bends 60 on the second to last graft segment

In the second to last graft segment, the unattached proximal bends 60 may reside as illustrated in Figure 39b, or it may be extended all the way to or near the proximal end of the graft, such as illustrated in Figure 39c.

### Enhanced Deployment Catheter

Referring to Figures 40 and 41, there is illustrated a fragmentary side elevational view of an enhanced flexibility embodiment of the deployment catheter of the present invention. Like numbers are used to refer to parts similar to those of Figure 25-28. In this embodiment, the distal component 135 of the central tubular core 132 comprises a flexible wall such as a braided polyimide tubing. In one embodiment, the polyimide tubing has an inside diameter of about 1.499 mm (0.059") and an outside diameter of about 1.803 mm (0.071"). An internal braid is made from 0.0381 mm (0.0015") stainless steel 304 wire at a pic count of about 20 braids per cm (50 braids per inch), such as may be obtained from Phelps Dodge (GA) or H.V. Technologies (GA). The use of flexible tubing such as spiral cut layers or woven or braided tubing in place of conventional stainless steel or other metal hypotubing increases the lateral flexibility of the assembled device, which facilitates the placement and deployment steps.

However, introduction of a flexible hypotube 135 creates a flex point in the catheter at about the junction 131 between the distal end 129 of outer sheath 128 and the proximal end of the outer tubular housing 138. To prevent kinking at the junction 131, a reinforcement structure 161 is preferably provided within the catheter, spanning the junction 131. In the illustrated embodiment, the reinforcement structure 161 is carried by the tubular extension 160 of intermediate tube 130. The reinforcement structure 161 is in the form of a tubular element such as a stainless steel hypotube. The illustrated hypotube has a length within the range of from about 40 mm to about 60 mm, a wall thickness within the range of from about 0.0508 mm (0.002") to about 0.127 mm (0.005") and is secured immovably to the tubular extension 160. Any of a variety of other reinforcement structures 161 can also be used, such as spiral cut or woven or braided layers, polymeric tubing and the like, depending upon the desired performance characteristics. By positioning the reinforcement structure 161 at about the axial location of the junction 131, the flexibility characteristics of the catheter can be optimized, while permitting a highly flexible hypotube 134.

The dimensions of the reinforcement tube 161 can be varied, depending upon the desired performance characteristics of the catheter. For example, in the embodiment illustrated in part in Figure 40A, the reinforcement tube extends proximally at least as far as the proximal stop 159 which will be discussed. The reinforcement tube 161 may also extend distally as far as the distal stop 158. In the embodiment illustrated in Figure 40A, the reinforcement tube 161 extends distally beyond the proximal stop 159 for a length of about 12.2 cm (4.8 inches) The reinforcing sleeve has an inside diameter of about 1.829 mm (0.072 inches) and an outside diameter of about 1.95 mm (0.076) inches. Other dimensions may be utilized, depending upon the desired balance between flexibility and kink resistance, as well as other performance characteristics. See, e.g., Figure 40

The braided polyimide hypotube 135, or other braided or woven reinforced tubular element can be secured to the enlarged diameter proximal component 134 of tubular core 132 (see Fig. 41) in any of a variety of ways. In the illustrated embodiment, a threaded insert 163 is adhesively bonded to the polyimide hypotube component 134 of the tubular core 132 using a flexible epoxy such as 310 T manufactured by Epotech (MASS.) or other adhesives known in the art.

A further optional feature of the deployment system in accordance with the present invention is illustrated in Figure 40A. In this simplified fragmentary view, the distal end of the intermediate tube 130 is illustrated as extending out of the distal end 129 of the outer sheath 128. A slit 167 is illustrated in the outer sheath, to accommodate the contralateral guidewire 166. The distal end of the intermediate tube 130 is provided with a proximal stop 159, for supporting the graft as has been discussed and for connecting the tubular extension 160 to the intermediate tube 130. The tubular extension 160 extends distally and supports the proximal end 165 of the ipsilateral tubular sheath 162.

In this embodiment, the proximal end 165 of ipsilateral tubular sheath 162 is tapered such as by necking down the outside diameter of the ipsilateral tubular sheath 162 for bonding to the tubular extension 160. This creates a generally conical space within the end of the tubular sheath 162, which can potentially collapse and cause binding upon distal advance of the outer sheath 128. Thus, a plug 177 having a generally conical shape may be provided to fill the proximal end 165 of the ipsilateral tubular sheath 162, thereby presenting a surface 167 for facing the graft (not illustrated). The plug 163 may be manufactured in any of a variety of ways, such as by injection molding or machining, or by introducing a curable or otherwise hardenable agent into the proximal end 165 and curing it in place to provide the surface 167.

Another optional feature of the deployment catheter is a spacer 179. In the embodiment illustrated in Figure 10, for example, it can be seen that the outside diameter of the ipsilateral tubular sheath 162 tapers down to approximately the inside diameter of the tubular extension 160, which is considerably smaller than the outside diameter of the intermediate tube 130. This low diameter space between the ipsilateral tubular sheath 162 and intermediate tube 130 creates an opportunity for the distal end of the outer sheath 128 to become engaged (snagged) with the proximal end 165 of sheath 162 as the outer sheath 128 is advanced distally along the deployment device. This may occur, for example, after the outer sheath 128 has been proximally retracted to release the contralateral graft, and thereafter distally advanced to support the ipsilateral graft during deployment of the contralateral graft.

To prevent the distal edge 129 of the outer sheath 128 from snagging on the proximal end 165 of the sheath 162, a spacer 179 is preferably positioned to fill the space between the stop 159 and the sheath 162. The spacer 179 may be a solid component, such as a molded or machined part, or a tubular element such as an extrusion. In one embodiment, as illustrated in Figure 40A, the spacer 179 comprises a molded tubular element having a diameter of about 4.699 mm (.185"), a total axial length of about 3.886 mm (.153"), and a length of about 18.8 mm (0.74") from 159 to the distal end of the spacer 179. A slot or recess 169 is provided for receiving the joint between the proximal end of the contralateral branch and the contralateral guidewire 166. The spacer 179 may be assembled as a separately manufactured component, or may be integrally formed with either the stop 159, the intermediate tube 130 or the sheath 162.

### Polymeric Sleeve or Membrane

An alternative, low profile linkage between adjacent segments may be provided by the polymeric sleeve or membrane. In this embodiment, any of the variations of the wire cage illustrated and described with respect to Fig.'s 3 - 12C and 34A-39C, may be coated on the inside, the outside, or preferably, on both the inside and the outside, by a polymeric sleeve, preferably of a laminated structure, which creates a flexible polymeric linkage of very low profile. In a preferred embodiment, where the wire cage is embedded between inner and outer layer(s) of polymeric material, the inner layer(s) may be adhered to the outer layer(s) through the openings between the adjacent wires of the support. The various mechanical linkages between adjacent segments of previously disclosed embodiments may be reduced in number or omitted when the embedding technology described below is used. Instead the ePTFE layer retains the desired spatial relationship between adjacent graft segments.

The sleeve or membrane that is used to cover the tubular wire graft cage can be manufactured from any of a variety of synthetic polymeric materials, or combinations thereof, including DACRON^{®}, polyester, polyethylene, polypropylene, fluoropolymers, polyurethane foamed films, silicon, nylon, silk, thin sheets of super-elastic materials, woven materials, polyethylene terephthalate (PET), or any other biocompatible material. In one embodiment of the present invention, the membrane material is a fluoropolymer, in particular, expanded polytetrafluoroethylene (ePTFE) having a node-fibril structure. The ePTFE membrane used in the present invention is manufactured from thin films of ePTFE that are each approximately 0.0025 to 0.025 mm in thickness. Thus, the films could be 0.0025, 0.0050, 0.0075, 0.0100, 0.0125, 0.0150, 0.0175, 0.0200, 0.0225, and 0.0250 mm thick.

From 1 to about 200 plies (layers) of ePTFE film may be stacked up and laminated to one another to obtain a membrane with the desired mechanical and structural properties. An even number of layers are preferably stacked together (e.g., 2, 4, 6, 8, 10, etc.), with approximately 2 to 20 layers being desirable. Cross-lamination occurs by placing superimposed sheets on one another such that the film drawing direction, or stretching direction, of each sheet is angularly offset by angles between 0 degrees and 180 degrees from adjacent layers or plies. Because the base ePTFE is thin, as thin as 0.0025 mm thick, superimposed films can be rotated relative to one another to improve mechanical properties of the membrane. In one preferred embodiment, the membrane is manufactured by laminating between 4 to 8 plies of ePTFE film, each film ply being about 0.0125 mm thick.

In this embodiment, the membrane is made by laminating 4 plies of ePTFE film, each film being about 0.0125 mm thick. The laminated ePTFE sheets are then sintered together at temperatures of about 370° C, under vacuum to adhere the film layers to one another. The resultant 8-ply laminate structure is typically 0.0375 mm thick. Additional details and variations on the ePTFE laminating technology are disclosed in U.S. Patent No. 5,925,075 to Myers et al.

Any of the variations of formed wire configurations disclosed herein, particularly those described with reference to Figs. 42A-E, may be coated on the lumenal (inner) and/or external surface, or embedded within a laminated ePTFE membrane in accordance with the present invention. For example, as shown in Fig. 42A, separate zig-zag segments, similar to those illustrated in Fig. 3, but lacking the connector 66, may be positioned at a substantially fixed axial distance from one another by embedding in an ePTFE membrane. In the Fig. 42A variation, the adjacent segments are rotationally positioned with respect to each other so that the proximal bends 60 from one segment align with the distal bends 62 from the adjacent segment. Thus, the axial length of a graft or graft portion formed by two adjacent segments is greater than or equal to the sum of the axial length of each individual segment.

The axial compressability, radial strength, and lateral flexibility of a graft utilizing the structure illustrated in Fig. 42A will be influenced by the various factors discussed previously herein, as well as by the spacing in an axial direction between adjacent proximal bends 60 and corresponding distal bends 62 on the adjacent segment. For example, as the axial spacing is increased, greater lateral flexibility may be achieved. However, axial compression of the graft may occur at a lower compressive force level, depending upon the structural integrity of the embedded PTFE wall. Specific axial spacings may be optimized for particular applications, depending upon the desired performance. In general, an axial separation between each proximal bend 60 and corresponding distal bend 62 will be within the range of from about 0 to about 3mm. Preferably, the spacing in a straight segment graft utilizing a wire diameter of about 0.356 mm (0.014") will be within the range of from about 0.5mm to about 1.5mm.

In another variation, shown in Fig. 42B, the separate zig-zag segments are rotationally aligned. In the illustrated configuration, adjacent segments 54 are nested within adjacent segments, such that each proximal bend 60 from a distal segment lies within angle α between two distal bends 62 of a proximal segment. In this embodiment, the axial length formed by two adjacent segments is less than the sum of the axial length 140 of each individual segment. This design may be beneficial in applications where greater radial support is desired.

Alternatively, a first segment 142 may be spaced axially apart from a second segment 144, such that the axial distance between a distal bend 62 on first segment 142 and distal bend 62 on second segment 144 exceeds the axial length 140 of the segment. Axial distances between two adjoining segments 142 and 144 may vary within the range of from about 100% to about 200% of the length 140 of the adjacent segment 142, depending upon the desired radial force and column strength of the resulting graft.

In the embodiment illustrated in Fig. 42B, ten segments are illustrated in a nested configuration, in which no interconnecting links are used to secure adjacent segments 54 to each other. Thus, the spatial relationship between adjacent segments 54 is maintained by the fabric or polymer layer or layers, to which the segments 54 are adhered and/or embedded.

In one nested embodiment having a 0.356 mm (0.014") filament and the wall pattern illustrated in Fig. 42B, the circumferential distance 148 between any pair of adjacent distal bends 62 or proximal bends 60 is about 20.8mm (0.82"). The axial distance 146 between a proximal bend 60 and adjacent distal bends 62 is approximately 19.6 mm (0.77"). The axial length of the first leg 124 and second leg 126 between apex 122 and first bend 128 or second bend 130 is approximately 2.54 mm (0.10"). See Figure 42E.

An alternative design is illustrated in Fig. 42C. In this variation of the wire support, a single length of wire or laser cut filament from sheet stock is formed into a zig-zag pattern which is adapted to be rolled to form a spiral configuration, such as disclosed by An et al., U.S. Patent No. 5,545,211. Unlike An, however, axially adjacent apexes in the wire support do not need to be interlinked. Thus, the present embodiment may be constructed without interlinking axially adjacent apexes as disclosed, for example, in U.S. Patent No. 5,217,483 to Tower. The resulting wire cage design is thus similar to that disclosed in U.S. Patent No. 5,665,115 to Cragg, after deletion of the loop members 12. Deletion of loop members 12 from the Cragg design is enabled by embedding the wire cage of the present invention in the multi-layer ePTFE or other polymeric membrane as disclosed herein.

### Flat Sheet or Tube Designs

Although previous embodiments have been described primarily in the context of formed wire, the embodiments of Figs. 42A-E may conveniently be formed from a flat sheet or tube of material such as Elgiloy, Nitinol, or other material having desired physical properties. Sheets having a thickness of no more than about 0.635 mm (0.025") and, preferably, no more than about 0.381 mm (0.015") are useful for this purpose. In one embodiment, the support structure is formed by laser cutting the appropriate pattern on a 0.356 mm (0.014") thickness Elgiloy foil or tube. Similarly, any of the other embodiments disclosed previously herein can be manufactured by laser cutting, chemical etching, or otherwise forming the wire cage support from a flat sheet or tube of Elgiloy or other suitable material.

One advantage of forming the wire cage such as by laser cutting is the ability to more precisely control the cross-sectional area of the filament at different points in the structure. For example, filament crossover points can be readily manufactured having only a single filament thickness, compared to a double filament thickness where the crossover is accomplished in a wire structure. Referring to Fig. 42D, there is illustrated a side wall pattern for a self-expandable vascular graft in which each segment 54 comprises a plurality of diamond-shaped cells 152 each having a proximal bend 60 and a distal bend 62. At junction 150, the radial wall thickness of the support structure would be two filament thicknesses if this structure were constructed from preformed wire. However, by cutting the structure of Fig. 42D from a solid walled tube or thin film sheet, the junction 150 may have the same thickness as any other portion of the filament, thereby minimizing the profile of the resulting graft. Adjacent segments 54 may be held with respect to each other by a polymeric layer such as PTFE, as is described elsewhere herein.

Another example of design flexibility which can be achieved using the laser cutting technique of the present invention is illustrated in Figure 42E. Referring to Fig. 42E, a detailed view is illustrated of either a proximal bend 60 or distal bend 62. In any embodiment having only a few or no interconnecting links between adjacent segments 54, the radial strength in the finished product will tend to be lower than the radial strength of a product with multiple interconnecting links between adjacent segments 54 in an otherwise comparable graft. As an alternate or supplement to adding interconnecting links between adjacent segments 54, the cross-sectional area of the filament 132 may be varied to affect the radial strength.

For example, the plan view of the filament 132 in the area of a bend 60 as seen in Fig. 42E has a first width in the relatively straight sections thereof, and a second, greater width in the bends, and a constant thickness throughout. In one embodiment, the bend 60 comprises an apex 122 in which the filament 132 has a width 134 of about 0.508 mm (0.020"). Each of a first leg 124 and second leg 126 has a width at at least one point of about 0.356 mm (0.014"). Thus, in the first and second leg sections, the transverse cross-section of the filament 132 is approximately square since, in this embodiment, it has been cut from a sheet having a thickness of 0.356 mm (0.014"). A first bend 128 and a second bend 130 each have a maximum width of approximately 0.508 mm (0.020"). The width in a bend is preferably at least about 110% and more preferably at least about 125% of the average width in the adjacent filament. Bend widths of greater than about 140% or 150% of the adjacent filament width may also be used. The foregoing values can be readily converted to cross sectional areas to apply the same concept where the filament enlargement in the area of a bend occurs in whole or in part in the radial instead of the circumferential direction.

By enlarging the cross-sectional area of the filament 132 in the area of apex 122, first bend 128 and second bend 130, particularly in the circumferential direction of the graft, the inventors have determined that the relative radial strength of the device can be increased while omitting or minimizing connecting links between adjacent segments 54. In this embodiment, the apex 122 has an outside radius of curvature of about 0.686 mm (0.027"), and an inside radius of curvature of about 0.279 mm (0.011"). The radius of curvature of the concave surface of the filament 132 in the area of first bend 128 or second bend 130 is approximately 3.81 mm (0.15"). The radius of the corresponding convex surface of each of the first bend 128 and second bend 130 is approximately 1.27 mm (0.05"). Any of the foregoing dimensions or radii can be varied considerably, within the scope of the present invention, to achieve particular physical property characteristics, as will be apparent to those of skill in the art.

If the foregoing floating segment embodiments exhibit inadequate column strength or radial strength, one or more links may be utilized to connect each adjacent pair of segments such as 142 and 144. Depending upon the degree of increased radial or column strength desired in the finished product, two or three or four or more links may be provided between each pair of segments 54. In one embodiment, at least one and as many as two or three or more links may be utilized for each adjacent pair of segments.

In addition, where links are desired between adjacent segments 54, links cut from a metal or other material tube or sheet can also be integrally formed with the adjacent segments 54 without adding wall thickness to the wire cage. See Fig. 42F.

### EXAMPLE 1

A Nitinol wire cage (See e.g., Fig.'s 1-3, 42A-D) is provided with both a lumenal (inner) covering and an exterior covering of ePTFE film. The lumenal and exterior coverings are both made from a uniaxially-oriented film having fibrils oriented substantially in a single direction wherein the fibrils are all substantially parallel to each other. The lumenal covering is provided with the fibrils oriented parallel to the longitudinal axis of the tubular stent; the exterior covering is provided with the fibrils oriented substantially circumferential to the tubular stent. The film used for both the lumenal and exterior coverings is a porous ePTFE film having a discontinuous, porous coating of a fluoroethylene polymer (FEP) adhesive applied to one side of the porous ePTFE film. Examination of the FEP coated side of the film by scanning electron microscopy reveals FEP on only small portions of the nodes and fibrils at the surface of the film. It is estimated that less than 10% of the available node and fibril surface area exposed at the surface of the film are covered by FEP. The presence of the FEP adhesive thus has little or no adverse effect on the porosity of the porous ePTFE film.

The FEP-coated porous ePTFE film is made by a process which comprises the steps of:
a) contacting a porous ePTFE film with another layer which is preferably a film of FEP or alternatively of another thermoplastic polymer;
b) heating the composition obtained in step a) to a temperature above the melting point of the thermoplastic polymer;
c) stretching the heated composition of step b) while maintaining the temperature above the melting point of the thermoplastic polymer; and
d) cooling the product of step c).

In addition to FEP, other thermoplastic polymers including thermoplastic fluoropolymers may also be used to make this coated film. The adhesive coating on the porous ePTFE film may be either continuous (non-porous) or discontinuous (porous) depending primarily on the amount and rate of stretching, the temperature during stretching, and the thickness of the adhesive prior to stretching.

The discontinuously FEP-coated porous ePTFE film used to construct this example is of about 0.01 mm thickness and has a density of about 0.3 g/cc. The microstructure of the porous ePTFE contains fibrils of about 50 micron mean fibril length.

A length of film approximately equal to the length of the support cage to be covered and having uniaxially-oriented fibrils is wrapped as a single layer around a hollow, tubular, 1.5 cm outside diameter mandrel of non-porous PTFE to form a seam. The seam edges overlap by about 3 mm. The fibrils of the film are oriented parallel to the longitudinal axis of the mandrel; the FEP-coated side of the film faces away from the surface of the mandrel. The wire support is carefully fitted over the film-wrapped portion of the mandrel. The length of the wire support is centered over the length of the film-wrapped mandrel. The wire support is then provided with an exterior covering of a tape of the film described above by wrapping the tape circumferentially around the exterior surface of the mandrel so that the edges of the circumferentially-wrapped tape overlap by about 3 mm to form the seam. The circumferentially wrapped covering is oriented so that the FEP-coated side of the tape faces inward in contact with the exterior surface of the wire support and the outward facing FEP-coated surface of the lumenal layer of film is exposed through the openings in the wire support. Except for the overlapped seam edges, the circumferentially-wrapped covering is only one film layer thick. The uniaxially-oriented fibrils of the microstructure of the circumferentially-wrapped tape are circumferentially-oriented about the exterior wire support surface.

The film-wrapped mandrel assembly is placed into an oven set at 360° C for a period of 4 minute after which the film-wrapped mandrel is removed from the oven and allowed to cool. Following cooling to approximately ambient temperature, the mandrel is removed from the film-wrapped wire support. The amount of heat applied is adequate to melt the FEP-coating on the porous ePTFE film and thereby cause adjacent layers of film to adhere to each other. Thus the lumenal layer of film is adhered to the exterior circumferentially wrapped layer through the openings between the adjacent wires of the support. The combined thickness of the lumenal and exterior coverings is about 0.025 mm.

### EXAMPLE 2

A stainless steel wire support of the type illustrated in Figure 42A or 42D and dimensioned for use in the human abdominal aorta is provided with a lumenal covering of a porous ePTFE film having a microstructure of biaxially-oriented fibrils. This is accomplished by wrapping a hollow tubular mandrel of non-porous PTFE with a layer of porous ePTFE film having a continuous (non-porous) coating of FEP with the FEP-coated side of the film facing outwardly away from the mandrel surface. This film is about 0.02 mm thick; the porous ePTFE has a microstructure of uniaxially-oriented fibrils with the fibrils oriented circumferentially about the exterior surface of the mandrel. The wire support is carefully fitted over the film-wrapped portion of the mandrel. The mandrel assembly is then placed into an oven set at 360° C for four minutes. After removal from the oven and subsequent cooling, the mandrel is removed from the support leaving the wrapped film adhered to the lumenal surface of the support. This film is then peeled from the lumenal support surface leaving the FEP-coating and some small shreds of residual porous ePTFE adhered to the lumenal surface of the support wires. By removing the film and leaving the FEP adhesive on the lumenal stent surface, the film serves only as a release substrate for the application of the adhesive to the support surface.

The mandrel is then provided with a single layer wrapping of a porous ePTFE film having a microstructure of biaxially-oriented fibrils. This film is of about 30 micron fibril length, about 0.08 mm thickness, about 0.3 g/cc density and does not have an FEP coating. The biaxially-oriented fibrils are oriented to be substantially parallel to the longitudinal axis of the mandrel and to the circumference of the mandrel.

The film is overlapped adequately to form a 2 mm wide, longitudinally oriented seamline parallel to the longitudinal axis of the mandrel. A sheet of polyamide film was temporarily placed over the surface of the seam and then connected with the surface of a hand-held iron set at 400° C to cause the PTFE film seam edges to adhere to each other. Excess material beyond the 2 mm wide seam is trimmed away and discarded. The support is again carefully fitted over the film-covered mandrel. The resulting assembly is placed into an oven set at 380° C for three minutes and then removed and allowed to cool, after which the mandrel is removed from the support. The porous ePTFE film appears to be well adhered to the lumenal surface of the wire support by the FEP coating left from the first, previously removed, layer of film. The wall thickness of the PTFE film covering is about 0.08 mm.

### EXAMPLE 3

A self expandable Nitinol wire support (e.g., Fig.'s 42A-42C) is adjusted from its collapsed outside diameter to an enlarged outside diameter by inserting a tapered stainless steel mandrel followed by a straight stainless steel mandrel. This support is then provided with a single layer exterior wrapping of the same discontinuously FEP-coated porous ePTFE coating used for the exterior wrapping of the support of Example 1. This is accomplished by wrapping the film about the exterior surface of the mandrel with the uniaxially-oriented fibrils of the film microstructure oriented parallel to the longitudinal axis of the support. This exterior covering is described as follows. A 2 mm wide seam is formed from the overlapped edges of the porous ePTFE film by temporarily placing a thin sheet of polyamide film over these edges and applying heat from a hand-held iron with a surface temperature of about 400° C. The film-wrapped support is then placed into an oven set at 380° C for 3 minutes, after which it is removed and allowed to cool. The film appears to be well adhered to the exterior surface of the support. The wall thickness of the film covering is about 0.01 mm. The enlarged support is then collapsed by the following process.

A series of 20 cm long 6-0 sutures are tied individually to each of the closed metal support openings adjacent to one end of the support. The film-covered support is provided with a temporary non-adhered additional wrapping of longitudinally-oriented film without FEP and having a microstructure of uniaxially-oriented fibrils. This temporary wrapping is intended as a dry lubricant. The enlarged support is then pulled by these sutures through a tapered die of round cross section and 2.5 cm length, the die having a tapered orifice with a 9.5 mm diameter bore at its entrance and a 4.5 mm diameter bore at its exit. The result is that the support is collapsed back to an outside diameter of 4.5 mm. The lubricity of the temporary covering of porous ePTFE film aids in making it possible to pull the support through the die. This temporary covering is removed after completion of the collapsing process. It is anticipated that the use of a tapered die having an approximately sized, smaller diameter exit bore would result in collapsing the support to its original collapsed diameter. The film-covered support is again enlarged to a diameter of 8 mm using a balloon catheter followed by a tapered stainless steel mandrel. The covering of porous ePTFE film appears to be fully intact after the collapsing and enlarging of the film-covered stent.

Support coverings may be affixed to a support surface by variations on this method. For example, a tubular sleeve may be made from a film of porous ePTFE and inverted back into itself and fitted over the inner and outer surfaces of a support. The inner and outer portions of the tubular sleeve may be thermally adhered to each other through the openings in the support wall, or may be adhered to the support surfaces by an adhesive such as FEP, or may be affixed to the support by suturing the open ends of the tube together.

### EXAMPLE 4

A formed wire (see e.g., Fig. 3 and Fig.'s 42A-C) made of Nitinol or any memory alloy may be constrained in a jig in its expanded layout and placed together with the jig into an oven, heated for at least two minutes, up to about one-half hour, at about 400° C to 600° C, e.g., 500° C, as described in U.S. Patent No. 5,879,366 to Shaw et al. The wire is cooled by air, water or any other means, and removed from the restraining jig. As the result of heating for 30 minutes at 500° C, the Nitinol wire obtains a "memory" for the particular configuration, which in this case is the enlarged tubular cage. Therefore, the wire exhibits super-elastic properties, which act to return the wire to the larger diameter tube even after extreme deformation, such as being collapsed within the delivery catheter. This memory-induced formed wire is then coated with one or more polymeric layers. The steps of coating the wire may occur either before the formed wire has been rolled into a tubular stent, or after the tubular stent has been formed.

The wire is coated with a bonding agent, for example FEP or other suitable thermoplastic polymer. If the wire is pre-rolled into a tube, a close tolerance FEP tube is slipped over the wire, so that the ends of the FEP tube are offset from the wire termination point. The FEP will then be heated and adhered to the wire during subsequent processing. The FEP coating can also be applied directly to the formed wire before rolling. Besides contacting the wire with a layer of FEP, the FEP coating may alternatively be applied by dipping, spraying, laminating between sheets, or any other means known in the art.

In one preferred method of applying an ePTFE membrane to a tubular, formed wire, a four-ply laminate is prepared from four tubular film layers (plies) of ePTFE. The film layers are placed onto a porous vacuum chuck with each film layer being rotated 90 degrees relative to one another.

One or more FEP coated formed wire structures are placed onto the four-ply laminate. Four additional layers of ePTFE film, rotated 90 degrees relative to each other, are placed onto the assembly, forming a second four-ply laminate structure, with the FEP coated wire structure(s) embedded between the two four-ply laminates.

This entire assembly is then covered with a Kapton tube or other high temperature plastic and placed into a sintering press. The sintering press applies vacuum through the porous vacuum chuck to the assembly. Sintering is conducted at temperatures of about 370° C for a period of several minutes, e.g., 15 minutes, up to several hours. The sintered assembly is cooled and the Kapton tube is removed and discarded.

Regardless of the particular configuration of wire cage (see e.g., Fig. 3 and Fig.'s 42A-C), where the wire is embedded in a polymeric sleeve or membrane, a cross section of the embedded support can be appreciated with reference to Fig. 43. Here, the wire 47 has a thin coating 48, which comprises a thermoplastic polymer adhesive, such as for example, FEP, described above. The coated wire is embedded within at least two layers of polymer film 20, such as for example, ePTFE, one each on the lumenal (inner) 30 and exterior 31 surfaces. The embedded support depicted in Fig. 43 is shown having two plies on each of the lumenal and external surfaces. As detailed above, however, preferably an even number of layers are stacked together (e.g., 2, 4, 6, 8, 10, etc.), with approximately 2 to 20 layers being desirable. Cross-lamination of superimposed sheets, angularly offset by angles between 0 degrees and 180 degrees from adjacent layers, is desired in order to improve mechanical properties of the membrane. As discussed in more detail above, the membrane is preferably manufactured by laminating between 4 to 8 plies of ePTFE film, each film ply being about 0.0125 mm thick. The laminated ePTFE sheets, fused together at temperatures of about 370° C under vacuum, form a low profile, flexible linkage between adjacent segments of the wire cage.

Other graft configurations and methods of coating wire stents with uniaxially and/or biaxially oriented ePTFE are encompassed by the present invention. One such alternate method is disclosed in U.S. Patent No. 5,788,626 to Thompson.

Another preferred embodiment of the present invention may be appreciated with reference to Fig.'s 44 A-C. In Fig. 44A, a two segment portion of the wire cage is shown in which the proximal 60 and distal 62 apexes from adjacent segments of the tubular member are aligned in the longitudinal axis, thereby essentially abutting one another as illustrated. In this variation, the wire cage is surrounded by a polymeric sleeve 44, preferably at least two ePTFE membranes, one along the lumenal surface and one along the exterior surface of the stent. The membranes may comprises any number of plies as discussed above. Alternatively, both surfaces of the cage may be covered by a single tube of ePTFE membrane (1-200 plys) extending through and folded over the wire cage resulting in a layer of ePTFE along both the lumenal and exterior surfaces. In either case, the PTFE envelope is spot welded in a pattern such as that shown in Fig. 44A, wherein the lumenal and exterior membranes are heat-fused in a plurality of spots 101. By spot-welding along the proximal and distal edges of the tubular member, the sleeve is closed around the support. Further, the pattern of four welding spots surrounding each apical junction, acts as a flexible linkage, thereby limiting movement of adjacent segments relative to one another. In areas of the graft in between the welding spots, the lumenal and exterior membranes are not necessarily fused.

As shown in end elevation from the distal margin of the graft (along line C-C) in Fig. 44C, the inner 30 and outer 31 membranes are fused at the welding spots 101, whereas the membrane layers can separate in between the welding spots. The sleeve is also illustrated surrounding the wire apexes 62. This design, like the previous variations in which the lumenal and exterior membranes were fused in all areas between adjacent wires, presents a very low profile, since the thickness of the PTFE membranes can be very small, as detailed above.

An alternative configuration is illustrated in Fig. 44B, wherein axially adjacent apexes of the support cage are circumferentially offset, such that distal apexes 62 from the adjacent segments are aligned with one another in the longitudinal axis (and proximal apexes 60 from adjacent segments are aligned with one another in the longitudinal axis). In this embodiment, the distal apex 62 from a proximal segment is oriented between two adjacent proximal apexes 60 of the distal segment. As in Fig. 44A, the tubular wire segments are surrounded on both sides by a polymeric sleeve 44. An identical pattern of welding spots 101 is used to fasten the inner and outer membranes at the axial ends of the tubular member. Thus, the thin end elevation wall profile (shown in Fig. 44C) for the graft variation shown in Fig. 44B would be the same as that presented by the graft of Fig. 44A. However, along the length of the graft, at the junctions between adjacent tubular wire segments, the pattern of welding spots is different than that shown in Fig. 44B. In the inside angle of each apex, the membranes are spot welded. Further, in a preferred variation, at least alternating distal and proximal apexes and preferably all apexes may be surrounded by several spot welds. The precise pattern is not critical as long as the spot welds serve the purpose of securing the inner and outer polymer layers together and minimizing movement between adjacent segments in the longitudinal (or axial) direction.

The spot welding of the two membrane layers may be accomplished by any spot heating means known in the art. For example, a pointed heating iron, like a conventional soldering iron, could be used as long as it was adapted to maintain a membrane temperature sufficient to bond the layers of polymer film together. Typically for ePTFE, a temperature of 370° for about 15 minutes would be sufficient to fuse the layers together. In one preferred method of spot welding the lumenal and exterior PTFE membranes, an RF chamber may be programmed to make all of the necessary welds at once.

In one preferred embodiment of the invention, the material of ePTFE membrane or sleeve is sufficiently porous to permit ingrowth of endothelial cells, thereby providing more secure anchorage of the prosthesis and potentially reducing flow resistance, sheer forces, and leakage of blood around the prosthesis. Porosity in polymeric sleeve materials may be estimated by measuring water permeability as a function of hydrostatic pressure, which will preferably range from about 20.7 to 41.4 kPa (3 to 6 psi).

The porosity characteristics of the polymeric sleeve may be either homogeneous throughout the axial length of the prosthesis, or may vary according to the axial position along the prosthesis. For example, referring to Fig.'s 1 and 2, different physical properties will be called upon at different axial positions along the prosthesis 42 in use. At least a proximal portion 55 and a distal portion 59 of the prosthesis 42 will seat against the native vessel wall, proximally and distally of the aneurysm. In these proximal and distal portions, the prosthesis preferably encourages endothelial growth, or, at least, permits endothelial growth to infiltrate portions of the prosthesis in order to enhance anchoring and minimize leakage. A central portion 57 of the prosthesis spans the aneurysm, and anchoring is less of an issue. Instead, maximizing lumen diameter and minimizing blood flow through the prosthesis wall become primary objectives. Thus, in a central zone 57 of the prosthesis 42, the polymeric membrane or sleeve 44 may either be nonporous, or provided with pores of relatively lower porosity

A multi-zoned prosthesis 42 may also be provided in accordance with the present invention by positioning a tubular sleeve 44 on a central portion 57 of the prosthesis, such that it spans the aneurysm to be treated, but leaving a proximal attachment zone 55 and a distal attachment zone 59 of the prosthesis 42 having exposed wires from the wire support 46. In this embodiment, the exposed wires 46 are positioned in contact with the vessel wall both proximally and distally of the aneurysm, such that the wire, over time, may become embedded in cell growth on the interior surface of the vessel wall.

In one embodiment of the prosthesis 42, the sleeve 44 and/or the wire support 46 is tapered, having a relatively larger expanded diameter at the proximal end 50 compared to the distal end 52. The tapered design may allow the prosthesis to conform better to the natural decreasing distal cross-section of the vessel, to reduce the risk of graft migration and potentially create better flow dynamics. The cage 46 can be provided with a proximal zone 55 and distal zone 59 that have a larger average expanded diameter than the central zone 57, as illustrated in Fig. 2. This configuration may desirably resist migration of the prosthesis within the vessel and reduce leakage around the ends of the prosthesis.

While a number of preferred embodiments of the invention and variations thereof have been described in detail, other modifications and methods of using and medical applications for the same will be apparent to those of skill in the art. Accordingly, it should be understood that various applications, modifications, and substitutions may be made of equivalents without departing from the scope of the claims.

## Claims

1. A tubular wire support (46) for combination with a sheath to produce a bifurcated endoluminal prosthesis, said tubular wire support comprising:
a main body support structure (200) having a proximal end (206), a distal end and a central lumen extending therethrough, the support structure comprising at least a first and second axially adjacent tubular segments (54), each segment comprising a plurality of wall struts (64) connected by proximal (60) and distal (62) bends;
a first branch support structure (202) having a proximal end, a distal end (208) and a central lumen therethrough, connected to the main body support structure;
a second branch support structure (204) having a proximal end, a distal end (210) and a central lumen extending therethrough; connected to the main body support structure;
at least two sliding links (220) inbetween the first and second segments; **characterised in that** the tubular wire support comprises at least one lock (221) on a wall strut (64) between a proximal bend and a distal bend for limiting axial movement of a sliding link along that strut.

2. The tubular wire support as in Claim 1 wherein the main body support structure and the first and second branch support structures are self-expandable from a radially collapsed state to a radially expanded state.

3. A tubular wire support as in Claim 2 wherein at least a portion of the tubular wire support has an expansion ratio of at least about 1:4.

4. The tubular wire support of Claim 1, further comprising a tubular sheath on the wire support.

5. The tubular wire support of Claim 4, wherein the sheath comprises a PTFE sleeve surrounding at least a central portion of the wire support.

6. the tubular wire support of Claim 1, wherein each segment comprises wire formed into a series of proximal bends, a series of distal bends, and a series of struts connecting the proximal and distal bends.

7. The tubular wire support of Claim 6, wherein each tubular segment comprises from about 4 proximal bends to about 12 proximal bends.

8. The tubular wire support of Claim 1, wherein the first and second branch support structures are pivotably attached to the main body support structure.

9. The tubular wire support of Claim 1, wherein the first and second sliding links join opposing proximal and distal bends on the first tubular segment and the second tubular segment.

10. The tubular wire support of Claim 1, comprising at least four sliding links between the first and second segments.

11. The tubular wire support of Claim 7, wherein at least some of the struts are substantially linear.

12. The tubular wire support of Claim 11, wherein the sliding link comprises a proximal bend or distal bend on the first segment slidably engaged with a strut on an adjacent segment.

13. The tubular wire support of Claim 1, wherein each of the at least two sliding links comprises a loop formed by a proximal or distal bend.

14. The tubular wire support of Claim 1, wherein the at least one lock comprises a loop (223) on the wall strut.

## Patentansprüche

1. Röhrenförmige Drahtstütze (46) zur Kombination mit einer Hülle, um eine gabelförmige endoluminale Prothese zu erzeugen, wobei die röhrenförmige Drahtstütze Folgendes umfasst:
eine Hauptkörperstützkonstruktion (200), die ein proximales Ende (206), ein distales Ende und ein zentrales Lumen, das sich dort hindurch erstreckt, aufweist, wobei die Stützkonstruktion mindestens ein erstes und zweites axial benachbartes röhrenförmiges Segment (54) umfasst, wobei jedes Segment eine Vielzahl von Wandstreben (64) umfasst, die durch proximale (60) und distale (62) Biegungen verbunden sind;
eine erste Verzweigungsstützkonstruktion (202), die ein proximales Ende, ein distales Ende (208) und ein zentrales Lumen, das sich dort hindurch erstreckt, aufweist, und mit der Hauptkörperstützkonstruktion verbunden ist;
eine zweite Verzweigungsstützkonstruktion (204), die ein proximales Ende, ein distales Ende (210) und ein zentrales Lumen, das sich dort hindurch erstreckt, aufweist, und mit der Hauptkörperstützkonstruktion verbunden ist;
mindestens zwei Gleitverbindungen (220) zwischen dem ersten und zweiten Segment; **dadurch gekennzeichnet, dass** die röhrenförmige Drahtstütze mindestens eine Verriegelung (221) an einer Wandstrebe (64) zwischen einer proximalen Biegung und einer distalen Biegung umfasst, um eine axiale Bewegung einer Gleitverbindung entlang dieser Strebe zu begrenzen.

2. Röhrenförmige Drahtstütze nach Anspruch 1, wobei sich die Hauptkörperstützkonstmktion und die erste und zweite Verzweigungsstützkonstruktion von einem radial zusammengezogenen Zustand in einen radial ausgedehnten Zustand selbst ausdehnen können.

3. Röhrenförmige Drahtstütze nach Anspruch 2, wobei mindestens ein Abschnitt der röhrenförmigen Drahtstütze ein Ausdehnungsverhältnis von mindestens etwa 1:4 aufweist.

4. Röhrenförmige Drahtstütze nach Anspruch 1, die des Weiteren eine röhrenförmige Hülle an der Drahtstütze umfasst.

5. Röhrenförmige Drahtstütze nach Anspruch 4, wobei die Hülle eine PTFE-Muffe umfasst, die mindestens einen zentralen Abschnitt der Drahtstütze umgibt.

6. Röhrenförmige Drahtstütze nach Anspruch 1, wobei jenes Segment Draht umfasst, der zu einer Reihe von proximalen Biegungen, einer Reihe von distalen Biegungen und einer Reihe von Streben, die die proximalen und distalen Biegungen verbinden, geformt ist.

7. Röhrenförmige Drahtstütze nach Anspruch 6, wobei jedes röhrenförmige Segment etwa 4 proximale Biegungen bis etwa 12 proximale Biegungen umfasst.

8. Röhrenförmige Drahtstütze nach Anspruch 1, wobei die erste und zweite Verzweigungsstützkonstruktion schwenkbar an der Hauptkörperstutzkonstruktion angebracht sind.

9. Röhrenförmige Drahtstütze nach Anspruch 1, wobei die erste und zweite Gleitverbindung gegenüberliegende proximale und distale Biegungen an dem ersten röhrenförmigen Segment und dem zweiten röhrenförmigen Segment verbinden.

10. Röhrenförmige Drahtstütze nach Anspruch 1, die mindestens vier Gleitverbindungen zwischen dem ersten und zweiten Segment umfasst.

11. Röhrenförmige Drahtstütze nach Anspruch 7, wobei mindestens einige der Streben im Wesentlichen linear sind.

12. Röhrenförmige Drahtstütze nach Anspruch 11, wobei die Gleitverbindung eine proximale Biegung oder eine distale Biegung an dem ersten Segment umfasst, die gleitfähig in eine Strebe an einem benachbarten Segment eingreift.

13. Röhrenförmige Drahtstütze nach Anspruch 1, wobei jede der mindestens zwei Gleitverbindungen eine Schlaufe umfasst, die von einer proximalen oder distalen Biegung gebildet ist.

14. Röhrenförmige Dralitstütze nach Anspruch 1, wobei die mindestens eine Verriegelung eine Schlaufe (223) an der Wandstrebe umfasst.

## Revendications

1. Renforcement métallique tubulaire (46) combiné à une gaine afin de produire une prothèse endoluminale bifurquée, ledit renforcement métallique tubulaire comprenant :
une structure de renforcement de corps principal (200) présentant une extrémité proximale (206), une extrémité distale et une lumière centrale s'étendant à l'intérieur, la structure de renforcement comprenant au moins des premier et deuxième segments tubulaires adjacent dans la direction axiale (54), chaque segment comprenant une pluralité de tiges pariétales (64) connectées par des coudes proximaux (60) et distaux (62) ;
une première structure de renforcement ramifiée (202) présentant une extrémité proximale, une extrémité distale (208) et une lumière centrale à l'intérieur, connectée à la structure de renforcement de corps principal ;
une deuxième structure de renforcement ramifiée (204) présentant une extrémité proximale, une extrémité distale (210) et une lumière centrale s'étendant à l'intérieur, connectée à la structure de renforcement de corps principal ;
au moins deux raccords coulissants (220) entre les premier et deuxième segments,
**caractérisé en ce que** le renforcement métallique tubulaire comprend au moins un dispositif de blocage (221) sur une tige pariétale (64) entre un coude proximal et un coude distal permettant de limiter le mouvement axial d'un raccord coulissant le long de cette tige.

2. Renforcement métallique tubulaire selon la revendication 1, dans lequel la structure de renforcement de corps principal et les première et deuxième structures de renforcement ramifiées sont auto-extensibles d'une position réduite dans la direction radiale à une position étendue dans la direction radiale.

3. Renforcement métallique tubulaire selon la revendication 2, dans lequel au moins une partie du renforcement métallique tubulaire présente un taux d'extension d'au moins 1:4 environ.

4. Renforcement métallique tubulaire selon la revendication 1, comprenant en outre une gaine tubulaire sur le renforcement métallique.

5. Renforcement métallique tubulaire selon la revendication 4, dans lequel la gaine se compose d'un manchon en PTFE entourant au moins une partie centrals du renforcement métallique.

6. Renforcement métallique tubulaire selon la revendication 1, dans lequel chaque segment se compose de fil métallique formant une série de coudes proximaux, une série de coudes distaux et une série de tiges connectant les coudes proximaux et distaux.

7. Renforcement tubulaire métallique selon la revendication 6, dans lequel chaque segment tubulaire se compose d'environ 4 coudes proximaux à environ 12 coudes proximaux.

8. Renforcement métallique tubulaire selon la revendication 1, dans lequel les première et deuxième structures de renforcement ramifiées sont connectées en pivot à la structure de renforcement de corps principal.

9. Renforcement métallique tubulaire selon la revendication 1, dans lequel les premier et deuxième raccords coulissants joignent des coudes proximaux et distaux opposés sur le premier segment tubulaire et le deuxième segment tubulaire.

10. Renforcement métallique tubulaire selon la revendication 1, comprenant au moins quatre raccords coulissants entre les premier et deuxième segments.

11. Renforcement métallique tubulaire selon la revendication 7, dans lequel au moins certaines des tiges sont essentiellement linéaires.

12. Renforcement métallique tubulaire selon la revendication 11, dans lequel le raccord coulissant se compose d'un coude proximal ou distal sur le premier segment entrant en prise coulissante avec une tige d'un segment adjacent.

13. Renforcement métallique tubulaire selon la revendication 1, dans lequel chacun des au moins deux raccords coulissants se compose d'une boucle formée par un coude proximal ou distal.

14. Renforcement métallique tubulaire selon la revendication 1, dans lequel l'au moins un dispositif de blocage se compose d'une boucle (223) sur la tige pariétale.
